# EUROPEAN PATENT SPECIFICATION

(11) **EP 3 936 127 B1**
(45) Date of publication and mention of the grant of the patent: **30.04.2025**
(21) Application number: 20765858.4
(22) Date of filing: 03.03.2020
(51) Int. Cl.: A61K 31/415, A61K 9/10, A61K 9/20, A61K 9/28, A61K 9/36, A61K 47/02, A61K 47/04, A61K 47/32, A61K 47/38, A61P 3/06, A61P 3/08, A61P 3/10, A61P 9/04, A61P 9/10, A61P 9/12, A61P 11/00, A61P 21/00, A61P 25/00, A61P 35/00, A61P 43/00, A61K 9/14

(54) **AMORPHOUS SOLID DISPERSION OF PYRAZOLE-AMIDE COMPOUND**
AMORPHE FESTE DISPERSION EINER PYRAZOLAMIDVERBINDUNG
DISPERSION SOLIDE AMORPHE D'UN COMPOSÉ PYRAZOLE-AMIDE

(30) Priority: 04.03.2019 JP 2019038327
(43) Date of publication of application: 12.01.2022
(73) Proprietor: Japan Tobacco Inc., Tokyo 105-6927 (JP)
(72) Inventor: NAKASHIMA, Yoshihiro, Takatsuki-shi, Osaka 569-1125 (JP); NAKASHIMA, Shun, Takatsuki-shi, Osaka 569-1125 (JP); MORI, Yoshimasa, Takatsuki-shi, Osaka 569-1125 (JP); NOZAWA, Satoshi, Takatsuki-shi, Osaka 569-1125 (JP); ISHIKAWA, Yoshiaki, Takatsuki-shi, Osaka 569-1125 (JP)
(74) Representative: Vossius & Partner Patentanwälte Rechtsanwälte mbB
(86) International application number: PCT/JP2020/008840
(87) International publication number: WO 2020/179770

(56) References cited:
- WO-A1-2018/021508
- JP-A- 2009 530 415
- JP-A- 2013 528 218
- JP-A- 2014 521 745

## Description

### [Technical Field]

The present invention relates to a solid dispersion containing an amorphous compound represented by the formula [I] : 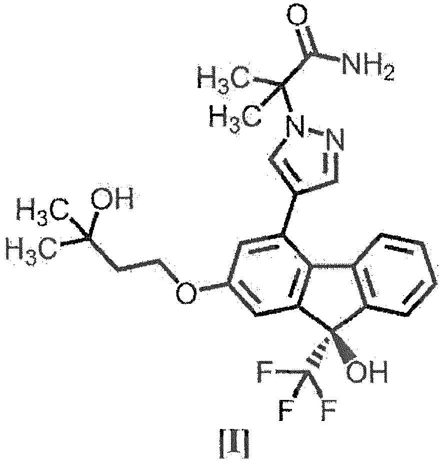 (hereinafter to be also referred to as the "compound of the formula [I]") or a pharmaceutically acceptable salt thereof, or a hydrate thereof, and a specific pharmaceutically acceptable polymer, and a manufacturing method thereof. In addition, the present invention also relates to a pharmaceutical composition containing the solid dispersion.

### [Background Art]

In the development of an orally administered pharmaceutical product, it is generally preferable that an active pharmaceutical ingredient (API) has high oral absorbability. When an active pharmaceutical ingredient is poorly soluble, the oral absorbability may be affected by meals. As a result, administration to patients who are unable to eat sufficiently may be limited. Even in patients who are able to eat, adherence may decrease since the timing of administration is limited. Known techniques to improve the solubility of poorly soluble compounds include salt formation, nanosizing, solid dispersion, solubilization using surfactant and cyclodextrin.

The compound of the formula [I], 2-{4-[(9R)-9-hydroxy-2-(3-hydroxy-3-methylbutyloxy)-9-(trifluoromethyl)-9H-fluoren-4-yl]-1H-pyrazol-1-yl}-2-methylpropanamide, a pharmaceutically acceptable salt thereof, and a monohydrate thereof are described in patent document 1 and patent document 2. Patent document 1 describes that the compound of the formula [I] and a monohydrate thereof have a pyruvate dehydrogenase kinase (PDHK) inhibitory activity and may become medicaments effective for the prophylaxis and/or treatment of diabetes, insulin resistance syndrome, metabolic syndrome, hyperglycemia, hyperlactacidemia, diabetic complications, cardiac failure, cardiomyopathy, myocardial ischemia, myocardial infarction, angina pectoris, dyslipidemia, atherosclerosis, peripheral arterial disease, intermittent claudication, chronic obstructive pulmonary diseases, brain ischemia, stroke, mitochondrial disease, mitochondrial encephalomyopathy, cancer or pulmonary hypertension. Patent document 2 describes a manufacturing method of the compound of the formula [I] or a pharmaceutically acceptable salt thereof, or a monohydrate thereof.

### [Document List]

### [Patent documents]

patent document 1: WO 2014/142290
patent document 2: WO 2018/021508

### [Summary of Invention]

### [Technical Problem]

The problem to be solved by the present invention is provision of a pharmaceutical composition containing a compound of the formula [I] with improved pharmacokinetics or a pharmaceutically acceptable salt thereof, or a hydrate thereof, and a manufacturing method thereof.

### [Solution to Problem]

The present inventors have found that an amorphous solid dispersion of a compound of the formula [I] or a pharmaceutically acceptable salt thereof, or a hydrate thereof can be obtained using a specific pharmaceutically acceptable polymer, and that the above-mentioned problem can be solved thereby, and completed the present invention.

### [Advantageous Effects of Invention]

According to the present invention, an amorphous form of a compound of the formula [I] or a pharmaceutically acceptable salt thereof, or a hydrate thereof with improved solubility as compared with crystals thereof can be provided. More specifically, according to the present invention, a solid dispersion capable of stably maintaining a compound of the formula [I] or a pharmaceutically acceptable salt thereof, or a hydrate thereof in an amorphous state is provided.

In some embodiments, precipitation of the amorphous solid dispersion of the present invention in an acidic solution is suppressed. Thus, the amorphous solid dispersion is not easily precipitated in the stomach when orally administered.

When a conventional tablet containing a crystal of a compound of the formula [I] or a pharmaceutically acceptable salt thereof, or a hydrate thereof is used, the oral absorbability of the compound of the formula [I] is influenced by the diet, and when administered under fasting conditions, the exposure may be reduced as compared with the administration after a meal. In contrast, an amorphous solid dispersion of the compound of the formula [I] according to the present invention shows high solubility in some embodiments regardless of the presence or absence of bile acid at the time of administration. Thus, it is less susceptible to the influence of the diet and shows high oral absorbability even when administered under fasting conditions.

### [Brief Description of Drawings]

Fig. 1 shows the dissolution profiles of a solid dispersion of a compound of the formula [I] prepared by a solvent method using hydroxypropylmethylcellulose acetate succinate (HPMCAS), methylcellulose or hypromellose, or a monohydrate crystal of the compound of the formula [I] in a pH 6.8 test media.
Fig. 2 shows the dissolution profiles of a solid dispersion of a compound of the formula [I] prepared by a hot-melt extrusion method using HPMCAS, methylcellulose or polyvinyl alcohol, or a monohydrate crystal of the compound of the formula [I] in a pH 6.8 test media.
Fig. 3 shows the precipitation behavior of a compound of the formula [I] in a pH 1.2 test media added with HPMCAS, copolyvidone, methylcellulose, hypromellose, or polyvinyl alcohol, or in a polymer-free pH 1.2 test media.
Fig. 4 shows the dissolution profiles of a solid dispersion tablet of a compound of the formula [I] obtained in Example 3 or 5 and a conventional tablet of a compound of the formula [I] in a pH 1.2, pH 4.0, pH 5.5, or pH 6.8 test media.
Fig. 5 shows the powder X-ray diffraction patterns of a solid dispersion tablet of a compound of the formula [I] obtained in Example 3 and a monohydrate crystal of a compound of the formula [I].
Fig. 6 shows the differential scanning calorimetry (DSC) thermograms of a solid dispersion tablet of a compound of the formula [I] obtained in Example 3 and a monohydrate crystal of a compound of the formula [I].
Fig. 7 shows the DSC thermograms of a solid dispersion tablet of a compound of the formula [I] obtained in Example 7 and a monohydrate crystal of a compound of the formula [I].
Fig. 8 shows the blood concentration profile of a compound of the formula [I] when the solid dispersion tablet obtained Example 3 or a conventional tablet obtained in Comparative Example 2 was orally administered to a dog.
Fig. 9 shows the blood concentration profile of a compound of the formula [I] when the solid dispersion tablet obtained Example 3 or Example 6 was orally administered to a dog.
Fig. 10 is a linear graph showing the blood concentration profile of a compound of the formula [I] when the conventional tablet obtained in Comparative Example 2 was orally administered to a human under fasting and fed conditions.
Fig. 11 is a semilog graph showing the blood concentration profile of a compound of the formula [I] when the conventional tablet obtained in Comparative Example 2 was orally administered to a human under fasting and fed conditions.
Fig. 12 is a linear graph showing the blood concentration profile of a compound of the formula [I] when the solid dispersion tablet obtained in Example 4 was orally administered to a human under fasting and fed conditions.
Fig. 13 is a semilog graph showing the blood concentration profile of a compound of the formula [I] when the solid dispersion tablet obtained in Example 4 was orally administered to a human under fasting and fed conditions.

### [Description of Embodiments]

The definitions of the terms in the present specification are as follows.

The "pharmaceutically acceptable salt" may be any salt known in the art that does not accompany excessive toxicity. Specifically, salts with inorganic acids, salts with organic acid, salts with inorganic bases, salts with organic bases
can be mentioned. Various forms of pharmaceutically acceptable salts are well known in the art and are described, for example, in the following reference documents:
(a) Berge et al., J. Pharm. Sci., 66, p1-19(1977),
(b) Stahl et al., "Handbook of Pharmaceutical Salts: Properties, Selection, and Use" (Wiley-VCH, Weinheim, Germany, 2002),
(c) Paulekuhn et al., J. Med. Chem., 50, p6665-6672 (2007).

Pharmaceutically acceptable salts of the compound of the formula [I] can be each obtained by reacting the compound of the formula [I] with an inorganic acid, an organic acid, an inorganic base or an organic base according to a method known per se. A pharmaceutically acceptable salt of the compound of the formula [I] may be formed as a half molecule, one molecule, or two or more molecules of acid or base with respect to one molecule of the compound of the formula [I].

Examples of the salt with inorganic acid include salts with hydrofluoric acid, hydrochloric acid, hydrobromic acid, hydroiodic acid, nitric acid, phosphoric acid and sulfuric acid.

Examples of the salt with organic acid include salts with acetic acid, adipic acid, alginic acid, 4-aminosalicylic acid, anhydromethylenecitric acid, benzoic acid, benzenesulfonic acid, calcium edetate, camphoric acid, 10-camphorsulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glycolylarsanilic acid, hexylresorcinic acid, hydroxy-naphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis (salicylic acid), galactaric acid, naphthalene-2-sulfonic acid, 2-naphthoic acid, 1,5-naphthalene disulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid and glutamic acid.

Examples of the salt with inorganic base include salts with lithium, sodium, potassium, magnesium, calcium, barium, aluminum, zinc, bismuth and ammonium.

Examples of the salt with organic base include salts with arecoline, betaine, choline, clemizole, ethylenediamine, N-methylglucamine, N-benzylphenethylamine, tris(hydroxymethyl)methylamine, arginine and lysine.

The compound of the formula [I] or a pharmaceutically acceptable salt thereof, or a monohydrate of the compound of the formula [I] can be synthesized by a known method, for example, the method described in the aforementioned patent document 1 or patent document 2.

The compound of the formula [I] or a pharmaceutically acceptable salt thereof may exist as a solvate thereof.

The "solvate" refers to the compound of the formula [I] or a pharmaceutically acceptable salt thereof with which a solvent molecule is coordinated, and also includes hydrates. Such solvates are preferably pharmaceutically acceptable solvates. Such solvates include, for example, hydrate, ethanol solvate, dimethylsulfoxide-solvate of the compound of the formula [I] or a pharmaceutically acceptable salt thereof. Specific examples include hemihydrate, monohydrate, dihydrate or mono ethanol solvate of the compound of the formula [I] or a monohydrate of the compound of the formula [I], 2/3 ethanol solvate of dihydrochloride of the same. Such solvates can be manufactured according to conventional methods.

The solvate is preferably a hydrate of the compound of the formula [I], more preferably a monohydrate of the compound of the formula [I], and is represented by the following structural formula [I-h]:

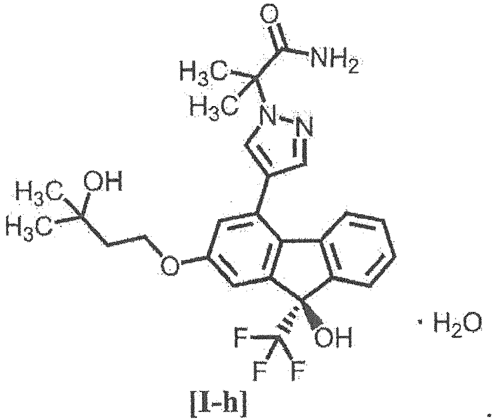

The "solid dispersion" means a mixture in which the active pharmaceutical ingredient (hereinafter to be also referred to as "API") is dispersed in a carrier, and is described, for example, in the following references:
(d) Chiou et al., Journal of Pharmaceutical Sciences., 60, p1281-1302(1971),
(e) Huang et al., Acta Pharmaceutica Sinica B., 4(1), p18-25(2014).

The "carrier" used in the preparation of a solid dispersion is a pharmaceutically acceptable polymer.

Examples of the "pharmaceutically acceptable polymer" include aminoalkylmethacrylate copolymer E, copolyvidone, ethylacrylate methylmethacrylate copolymer, hydroxypropylmethylcellulose acetate succinate (HPMCAS), hydroxypropylmethylcellulose phthalate, hypromellose, macrogol 6000, methylcellulose, ethylene gylcol and vinyl alcohol graft copolymer, polyoxyethylene(160) polyoxypropylene(30) glycol, polyvinyl alcohol-acrylic acid-methylmethacrylate copolymer, polyvinyl caprolactam-polyvinyl acetate-polyethylene glycol graft copolymer, povidone, polyvinyl alcohol. Preferred polymer is HPMCAS, methylcellulose, copolyvidone, hypromellose, or polyvinyl alcohol.

The "amorphous solid dispersion" means a solid dispersion in which most of the APIs contained in the above-mentioned solid dispersion are amorphous. In the present specification "mostly amorphous" means that not less than 90%, preferably not less than 95%, more preferably not less than 99%, of the compound of the formula [I] or a pharmaceutically acceptable salt thereof, or a hydrate thereof is amorphous. Unless particularly specified in the present specification, a solid dispersion means an amorphous solid dispersion.

That API is in an amorphous form can be confirmed, for example, by powder X-ray diffraction. When API is a crystal, peaks peculiar to API is generally observed by powder X-ray diffraction, whereas an amorphous form shows a halo pattern without the specific peak derived from API by powder X-ray diffraction in many cases.

That API is in an amorphous form can also be confirmed by DSC. When API is a crystal, specific peaks are generally observed due to changes in the crystal form, desorption of solvent from solvate, melting, whereas an amorphous form shows a halo pattern without such peaks in many cases.

The amorphous solid dispersion of the present invention can be used as a pharmaceutical composition as it is or in combination with a pharmaceutically acceptable carrier.

Examples of the "pharmaceutically acceptable carrier" include various organic or inorganic carrier substances conventionally used as preparation materials, for example, excipient, disintegrant, binder, fluidizer, lubricant, adsorbent, coating agent for solid preparations, and base, emulsifier, wetting agent, stabilizer, stabilizing agent, dispersing agent, plasticizer, pH modifier, absorption promoter, gelling agent, antiseptic, filler, dissolving agent, solubilizing agent, suspending agent for semisolid preparations. Where necessary, moreover, additives such as preservative, antioxidant, colorant, sweetening agent
may be used.

The pharmaceutical composition of the present invention can be in various dosage forms such as tablet, capsule, powder, granule, and can be manufactured by a conventional method. For example, a pharmaceutical preparation can be prepared through a formulation step such as mixing step, granulation step, tableting step, capsule filling step, coating step. A pharmaceutical preparation containing the amorphous solid dispersion of the present invention is preferably a tablet.

Examples of the "excipient" include lactose, lactose hydrate, sucrose, D-mannitol, D-sorbitol, cornstarch, dextrin, microcrystalline cellulose, crystalline cellulose, carmellose, carmellose calcium, sodium carboxymethyl starch, low-substituted hydroxypropyl cellulose, gum arabic, calcium silicate.

Examples of the "disintegrant" include carmellose, carmellose calcium, carmellose sodium, sodium carboxymethyl starch, croscarmellose sodium, crospovidone, low-substituted hydroxypropyl cellulose, hypromellose, crystalline cellulose, calcium silicate, silicified microcrystalline cellulose.

A preferred disintegrant is croscarmellose sodium, low-substituted hydroxypropyl cellulose, calcium silicate or silicified microcrystalline cellulose.

Examples of the "binder" include hydroxypropyl cellulose, hypromellose, povidone, crystalline cellulose, sucrose, dextrin, starch, gelatin, carmellose sodium, gum arabic.

Examples of the "fluidizer" include light anhydrous silicic acid, magnesium stearate.

Examples of the "lubricant" include magnesium stearate, calcium stearate, talc. A preferred lubricant is magnesium stearate.

Examples of the "base" include water, animal and vegetable oils (olive oil, corn oil, peanut oil, sesame oil, castor oil etc.), lower alcohols (ethanol, propanol, propylene glycol, 1,3-butyleneglycol, phenol etc.), higher fatty acid and ester thereof, waxes, higher alcohol, polyhydric alcohol, hydrocarbons (white petrolatum, liquid paraffin, paraffin etc.), hydrophilic petrolatum, purified lanolin, absorption ointment, hydrolyzed lanolin, hydrophilic ointment, starch, pullulan, gum arabic, gum tragacanth, gelatin, dextran, cellulose derivative (methylcellulose, carboxymethylcellulose, hydroxyethylcellulose, hydroxypropyl cellulose etc.), synthetic polymers (carboxyvinyl polymer, sodium polyacrylate, polyvinyl alcohol, polyvinylpyrrolidone etc.), propylene glycol, macrogol (macrogol 4000 etc.), titanium oxide, triacetin, and combinations of two or more kinds thereof.

Examples of the "preservative" include ethyl parahydroxybenzoate, chlorobutanol, benzyl alcohol, sodium dehydroacetate, sorbic acid.

Examples of the "antioxidant" include sodium sulfite, ascorbic acid.

Examples of the "colorant" include food colors (e.g., Food Color Red No. 2 or 3, Food Color yellow No. 4 or 5 etc.), β-carotene.

Examples of the "sweetening agent" include saccharin sodium, dipotassium glycyrrhizinate, aspartame.

Examples of the "adsorbent" include light anhydrous silicic acid, calcium silicate, microcrystalline cellulose.

A core tablet composed of the amorphous solid dispersion or a combination of the amorphous solid dispersion and a pharmaceutically acceptable carrier of the present invention may be coated. Examples of the coating include sugar coating, film coating.

Examples of the agent used for sugar coating include sucrose, erythritol, maltitol. These agents and the aforementioned pharmaceutically acceptable carrier may be used in combination for the coating.

Examples of the agent used for film coating include methylhydroxyethylcellulose, ethylcellulose, hydroxypropyl cellulose, povidone, carboxymethylcellulose sodium, polyethylene glycol, acrylic polymer, polyvinyl alcohol, hypromellose. These agents and the aforementioned pharmaceutically acceptable carrier may be used in combination for the coating. In addition, a coating agent mixed powder such as Kollicoat (registered trade mark, BASF), OPADRY (registered trade mark, Japan Colorcon) may also be used.

The "solid dispersion tablet" means a tablet formulated using the amorphous solid dispersion of the present invention. The "conventional tablet" means a tablet substantially free of the amorphous solid dispersion of the present invention.

The manufacturing method of the solid dispersion of the present invention is not particularly limited, and solvent method (precipitation, spray drying, freeze-drying, drying under reduced pressure), hot-melt extrusion method, cogrinding method, supercritical method can be mentioned. Among these, preferred is solvent method or hot-melt extrusion method.

The solvent method is a method in which API and a pharmaceutically acceptable polymer are dissolved or dispersed in a solvent, and then the solvent is evaporated. The solvent used for the solvent method is not particularly limited as long as it can dissolve or disperse API and the pharmaceutically acceptable polymer.

Examples of the solvent include water, dichloromethane, dichloroethane, chloroform, methanol, ethanol, propanol, isopropanol, acetone, methyl ethyl ketone, diethyl ether, dibutyl ether, n-hexane, cyclohexane, n-heptane, benzene, toluene, xylene, acetic acid, propionic acid, ethyl acetate, dimethylformamide, dimethylacetamide. These solvents may be used alone or as a mixed solvent. A preferred solvent is ethanol or acetone. The solvent can be removed, for example, by drying by heating or drying under reduced pressure.

The hot-melt extrusion method is a process of continuously heating, kneading of the starting materials such as API, pharmaceutically acceptable polymer, and extruding the resulting molten material through a temperature controlled extruder. The melting can be performed at, for example, 120°C - 200°C (preferably, 125°C - 175°C). A general extruder is equipped with barrels and screws in the inside thereof. As the extruder, a twin-screw extruder having two screws is preferred.

For the solid dispersion of the present invention, solid dispersion particles having any particle size can be easily obtained by milling with an appropriate pulverizer.

A pharmaceutical preparation composed of a solid dispersion obtained by the manufacturing method of the present invention, or a pharmaceutical preparation containing a solid dispersion can be safely administered to mammals (e.g., rat, mouse, guinea pig, monkey, bovine, dog, swine, human etc.) orally or parenterally (e.g., intravenously, intramuscular, subcutaneous, intraorgan, intranasal, intradermal, instillation, intracerebral, intrarectal, vaginal, or intraperitoneal administration, or directly to the lesion). The dose of a compound of the formula [I] or a pharmaceutically acceptable salt thereof or a hydrate thereof contained in a pharmaceutical preparation composed of a solid dispersion obtained by the manufacturing method of the present invention, or a pharmaceutical preparation containing the solid dispersion varies depending on the subject of administration, administration route, target disease, symptoms.
For example, for oral administration to an adult patient (body weight about 60 kg), a single dose is generally about 0.02 - about 30 mg/kg body weight, preferably about 0.2 - about 20 mg/kg body weight, further preferably about 0.5 - about 10 mg/kg body weight, and this amount is desirably administered once or several times (e.g., three times) per day.

A preferred embodiment of the present invention is described below.

API in the solid dispersion of the present invention is an amorphous compound of the formula [I], 2-{4-[(9R)-9-hydroxy-2-(3-hydroxy-3-methylbutyloxy)-9-(trifluoromethyl)-9H-fluoren-4-yl]-1H-pyrazol-1-yl}-2-methylpropanamide or a pharmaceutically acceptable salt thereof or a monohydrate thereof.

The content of a compound of the formula [I] in the pharmaceutical composition (pharmaceutical preparation) of the present invention varies depending on the dosage form and administration route. For example, it is 0.5 - 50 mass % in the case of a pharmaceutical preparation for oral administration.

As a carrier constituting the solid dispersion of the present invention, the aforementioned pharmaceutically acceptable polymers can be mentioned. Among those, preferred are one to four kinds selected from the group consisting of HPMCAS, methylcellulose, hypromellose and polyvinyl alcohol, more preferred is HPMCAS, a mixture of HPMCAS and methylcellulose, or a mixture of HPMCAS, methylcellulose and polyvinyl alcohol.

In the present specification, the "weight ratio of x and y" or "mixing weight ratio of x and y" is shown by "weight of x:weight of y".

When the solid dispersion of the present invention contains HPMCAS, the weight ratio of a compound of the formula [I] or a pharmaceutically acceptable salt thereof or a hydrate thereof, and HPMCAS in the solid dispersion is within the range of from 1:0.01 to 1:20, preferably within the range of from 1:0.05 to 1:20, more preferably from 1:0.1 to 1:10, further preferably from 1:0.15 to 1:2, particularly preferably from 1:0.4 to 1:0.6.

When the solid dispersion of the present invention contains methylcellulose, the weight ratio of a compound of the formula [I] or a pharmaceutically acceptable salt thereof or a hydrate thereof, and methylcellulose in the solid dispersion is preferably within the range of from 1:0.01 to 1:5, more preferably from 1:0.03 to 1:2, further preferably from 1:0.05 to 1:1, particularly preferably from 1:0.08 to 1:0.12.

When the solid dispersion of the present invention contains polyvinyl alcohol, the weight ratio of a compound of the formula [I] or a pharmaceutically acceptable salt thereof or a hydrate thereof, and polyvinyl alcohol in the solid dispersion is preferably within the range of from 1:0.05 to 1:20, more preferably from 1:0.1 to 1:10, further preferably from 1:0.2 to 1:2, particularly preferably from 1:0.4 to 1:0.6.

A specific preferred embodiment of the solid dispersion of the present invention is an amorphous solid dispersion of a compound of the formula [I], containing
(1) a compound of the formula [I] or a pharmaceutically acceptable salt thereof or a hydrate thereof, and
(2) HPMCAS.

In this embodiment, the weight ratio of a compound of the formula [I] or a pharmaceutically acceptable salt thereof or a hydrate thereof, and HPMCAS is preferably within the range of from 1:0.05 to 1:20. This range is more preferably from 1:0.1 to 1:10, further preferably from 1:0.15 to 1:2, particularly preferably from 1:0.4 to 1:0.6.

A specific more preferred embodiment of the solid dispersion of the present invention is an amorphous solid dispersion of a compound of the formula [I] containing
(1) a compound of the formula [I] or a pharmaceutically acceptable salt thereof or a hydrate thereof, and
(2) HPMCAS and methylcellulose.

In this embodiment, a preferable weight ratio of a compound of the formula [I] or a pharmaceutically acceptable salt thereof or a hydrate thereof, and HPMCAS is as described above.

In this embodiment, the weight ratio of a compound of the formula [I] or a pharmaceutically acceptable salt thereof or a hydrate thereof, and methylcellulose is preferably within the range of from 1:0.01 to 1:5. This range is more preferably from 1:0.03 to 1:2, further preferably from 1:0.05 to 1:1, particularly preferably from 1:0.08 to 1:0.12.

Another specific more preferred embodiment of the solid dispersion of the present invention is an amorphous solid dispersion of a compound of the formula [I] containing
(1) a compound of the formula [I] or a pharmaceutically acceptable salt thereof or a hydrate thereof, and
(2) HPMCAS, methylcellulose and polyvinyl alcohol.

In this embodiment, the weight ratio of a compound of the formula [I] or a pharmaceutically acceptable salt thereof or a hydrate thereof, and HPMCAS is preferably within the range of from 1:0.01 to 1:5. This range is more preferably from 1:0.03 to 1:1, further preferably from 1:0.05 to 1:0.5, particularly preferably from 1:0.07 to 1:0.13.

In this embodiment, a preferable weight ratio of a compound of the formula [I] or a pharmaceutically acceptable salt thereof or a hydrate thereof, and methylcellulose is as described above.

In this embodiment, the weight ratio of a compound of the formula [I] or a pharmaceutically acceptable salt thereof or a hydrate thereof, and polyvinyl alcohol is preferably within the range of from 1:0.05 to 1:20. This range is more preferably from 1:0.1 to 1:10, further preferably from 1:0.2 to 1:2, particularly preferably from 1:0.4 to 1:0.6.

### [Example]

While the present invention is specifically explained in the following by referring to Examples, the present invention is not limited to these Examples. The mixing ratio of each component in the following examples may be increased or decreased by about 20%, preferably about 10%. The monohydrate of the compound of the formula [I] used in the following Examples and Comparative Examples was synthesized according to the method described in the aforementioned patent document 2. As a pharmaceutically acceptable polymer and a pharmaceutically acceptable carrier, the 17th revision of the Japanese Pharmacopoeia or the Japanese Pharmaceutical Excipients 2018 compatible product was used.

### Example 1

### (1) Preparation of solid dispersion granule using HPMCAS as carrier (preparation by solvent method)

A monohydrate of the compound of the formula [I] (0.52 g) and HPMCAS (trade name: AQOAT AS-LF, manufactured by Shin-Etsu Chemical Co., Ltd.) (0.25 g) were dissolved in acetone (1.5 g). The obtained solution was dried overnight by a vacuum dryer (trade name: DRV320DA, manufactured by ADVANTEC) set at 60°C, and then sieved through a sieve with an opening of 180 µm to give the solid dispersion granules.

### (2) Preparation of solid dispersion granule using methylcellulose as carrier (preparation by solvent method)

A monohydrate (0.52 g) of the compound of the formula [I] and methylcellulose (trade name: METOLOSE SM-4, manufactured by Shin-Etsu Chemical Co., Ltd.) (0.25 g) were dissolved in a mixture (1.9 g) of ethanol: water = 8:2. The obtained solution was dried overnight by a vacuum dryer (trade name: DRV320DA, manufactured by ADVANTEC) set at 60°C, and then sieved through a sieve with an opening of 180 µm to give the solid dispersion granules.

### (3) Preparation of solid dispersion granule using hypromellose as carrier (preparation by solvent method)

A monohydrate (0.52 g) of the compound of the formula [I] and hypromellose (trade name: TC-5E, manufactured by Shin-Etsu Chemical Co., Ltd.) (0.25 g) were dissolved in a mixture (1.9 g) of ethanol: water = 8:2. The obtained solution was dried overnight by a vacuum dryer (trade name: DRV320DA, manufactured by ADVANTEC) set at 60°C, and then sieved through a sieve with an opening of 180 µm to give the solid dispersion granules.

### Example 2

### (1) Preparation of solid dispersion granule using HPMCAS as carrier (preparation by hot-melt extrusion method)

A monohydrate (4.1 g) of the compound of the formula [I] and HPMCAS (trade name: AQOAT AS-LMP, manufactured by Shin-Etsu Chemical Co., Ltd.) (2.0 g) were mixed in a glass container. The mixing powder (5 g) was processed for 5 min with a twin screw extruder (trade name: HAKKE MiniCTW, manufactured by Thermo Fisher Scientific) at a kneading unit barrel temperature 150°C and screw speed 100 rpm to give a cylinder-shaped moldings. The obtained moldings were milled by a powermill (trade name: new PowerMill, manufactured by OSAKA CHEMICAL Co., Ltd.) at rotating speed 22,000 rpm to give the solid dispersion granules.

### (2) Preparation of solid dispersion granule using methylcellulose as carrier (preparation by hot-melt extrusion method)

A monohydrate (4.1 g) of the compound of the formula [I] and methylcellulose (trade name: METOLOSE SM-4, manufactured by Shin-Etsu Chemical Co., Ltd.) (2.0 g) were mixed in a glass container. This mixed powder (5 g) was processed for 5 min with a twin screw extruder (trade name: HAKKE MiniCTW, manufactured by Thermo Fisher Scientific) at a kneading unit barrel temperature 150°C and screw speed 100 rpm to give a cylinder-shaped moldings. The obtained moldings were milled by a PowerMill (trade name: new PowerMill, manufactured by OSAKA CHEMICAL Co., Ltd.) at rotating speed 22,000 rpm to give the solid dispersion granules.

### (3) Preparation of solid dispersion granule using hypromellose as carrier (preparation by hot-melt extrusion method)

A monohydrate (4.1 g) of the compound of the formula [I] and hypromellose (trade name: TC-5E, manufactured by Shin-Etsu Chemical Co., Ltd.) (2.0 g) were mixed in a glass container. This mixed powder (5 g) was processed for 5 min with a twin screw extruder (trade name: HAKKE MiniCTW, manufactured by Thermo Fisher Scientific) at a kneading unit barrel temperature 180°C and screw speed 100 rpm to give a cylinder-shaped moldings. The obtained moldings were milled by a powermill (trade name: new PowerMill, manufactured by OSAKA CHEMICAL Co., Ltd.) at rotating speed 22,000 rpm to give the solid dispersion granules.

### (4) Preparation of solid dispersion granule using polyvinyl alcohol as carrier (preparation by hot-melt extrusion method)

A monohydrate (4.1 g) of the compound of the formula [I] and polyvinyl alcohol (trade name: JL-05E, manufactured by JAPAN VAM & POVAL CO., LTD.) (2.0 g) were mixed in a glass container. This mixed powder (5 g) was processed for 5 min with a twin screw extruder (trade name: HAKKE MiniCTW, manufactured by Thermo Fisher Scientific) at a kneading unit barrel temperature 180°C and screw speed 100 rpm to give a cylinder-shaped moldings. The obtained moldings were milled by a powermill (trade name: new PowerMill, manufactured by OSAKA CHEMICAL Co., Ltd.) at rotating speed 22,000 rpm to give the solid dispersion granules.

### Example 3

### Preparation of solid dispersion tablet using HPMCAS and methylcellulose as carrier (preparation by solvent method (middle-scale))

A monohydrate (259 g) of the compound of the formula [I] and HPMCAS (trade name: AQOAT AS-LG, manufactured by Shin-Etsu Chemical Co., Ltd.) (125 g) were dissolved in acetone (625 g). Methylcellulose (trade name: METOLOSE SM-4, manufactured by Shin-Etsu Chemical Co., Ltd.) (25 g) was added thereto and the mixture was stirred for 30 min or longer. This solution was added to a mixture of calcium silicate (trade name: Florite RE, manufactured by Tomita Pharmaceutical Co., Ltd.) (100 g), light anhydrous silicic acid (trade name: Aerosil 200, manufactured by NIPPON AEROSIL) (150 g) and croscarmellose sodium (trade name: Ac-Di-Sol, manufactured by FMC Health and Nutrition) (75 g), and granulated by a high-shear granulator (trade name: FM-VG-10, manufactured by POWREX). The obtained granules were dried by a vacuum dryer (trade name: VOD-4, manufactured by IKEDA SCIENTIFIC Co., Ltd.) set at 80°C until the residual acetone concentration became not more than 2.0%, and then milled through a screen mill (trade name: QC-U5, manufactured by Quadro Engineering) with an opening of 610 µm. This operation was repeated twice, and the combined granules (1174 g) were blended with magnesium stearate (trade name: Parteck LUB MST, manufactured by Merck) (5 g) and then compressed to give a core tablet (mass 295.0 mg, hardness about 100N, caplet form (13.7x6.3 mm)). The obtained core tablets (885 g) were coated with a water dispersion containing a mixture (trade name: OPADRY, manufactured by Japan Colorcon) (36 g) of polyvinyl alcohol, titanium oxide, macrogol, and talc in a coating machine (trade name: HCT-LABO, manufactured by Freund) to give the tablet containing 100 mg of the compound of the formula [I] per tablet.

### Example 4

### Preparation of solid dispersion tablet using HPMCAS and methylcellulose as carrier (preparation by solvent method (large-scale))

A monohydrate (259 g) of the compound of the formula [I] and HPMCAS (trade name: AQOAT AS-LG, manufactured by Shin-Etsu Chemical Co., Ltd.) (125 g) were dissolved in acetone (625 g). Methylcellulose (trade name: METOLOSE SM-4, manufactured by Shin-Etsu Chemical Co., Ltd.) (25 g) was added thereto and the mixture was stirred for 30 min or longer. This solution was added to a mixture of calcium silicate (trade name: Florite RE, manufactured by Tomita Pharmaceutical Co., Ltd.) (100 g), light anhydrous silicic acid (trade name: Aerosil 200, manufactured by NIPPON AEROSIL) (150 g) and croscarmellose sodium (trade name: Ac-Di-Sol, manufactured by FMC Health and Nutrition) (75 g), and granulated by a high-shear granulator (trade name: FM-VG-10, manufactured by POWREX). The granules were dried by a vacuum dryer (trade name: VOD-4, manufactured by IKEDA SCIENTIFIC Co., Ltd.) set at 80°C until the residual acetone concentration became not more than 2.0%, and then milled through a screen mill (trade name: QC-U10, manufactured by Quadro Engineering) with an opening of 610 µm to give solid dispersion granules. This operation was repeated 8 times, and the combined granules (10498 g) were blended with magnesium stearate (trade name: Parteck LUB MST, manufactured by Merck) (50 g) and then compressed to give a core tablet (mass 295.0 mg, diameter 9.0 mm). The obtained core tablets (1121 g) were coated with a water dispersion containing a mixture (trade name: OPADRY, manufactured by Japan Colorcon) (45 g) of polyvinyl alcohol, titanium oxide, macrogol, and talc in a coating machine (trade name: HCT-LABO, manufactured by Freund) to give the tablet containing 100 mg of the compound of the formula [I] per tablet.

### Example 5

### Preparation of solid dispersion tablet using HPMCAS, methylcellulose and polyvinyl alcohol as carrier (preparation by hot-melt extrusion method)

A monohydrate (20.7 g) of the compound of the formula [I], HPMCAS (trade name: AQOAT AS-LF, manufactured by Shin-Etsu Chemical Co., Ltd.) 2.4 g, methylcellulose 2.0 g (trade name: METOLOSE SM-4,manufactured by Shin-Etsu Chemical Co., Ltd.), polyvinyl alcohol (trade name: JL-05E, manufactured by JAPAN VAM & POVAL CO., LTD.) (9.6 g) and calcium silicate (trade name: Florire RE, manufactured by Tomita Pharmaceutical Co., Ltd.) (4.0 g) were mixed in a high-shear granulator (trade name: MECHANOMILL, manufactured by OKADA SEIKO CO., LTD.). This mixed powder (5 g) was processed with a twin screw extruder (trade name: HAKKE MiniCTW, manufactured by Thermo Fisher Scientific) at a kneading unit barrel temperature 160°C and screw speed 100 rpm to give a cylinder-shaped moldings. This operation was performed 7 times, and the combined moldings were milled by a powermill (trade name: new PowerMill, manufactured by OSAKA CHEMICAL Co., Ltd.) at rotating speed 22,000 rpm. The obtained milled granules (34 g) were mixed with low-substituted hydroxypropyl cellulose (trade name: L-HPC LH-B1, manufactured by Shin-Etsu Chemical Co., Ltd.) (8.7 g), silicified microcrystalline cellulose (trade name: PROSOLV SMCC 50, manufactured by JRS Pharma) (5.2 g) and magnesium stearate (trade name: Parteck LUB MST, manufactured by Merck) (0.2 g), and then compressed to give a core tablet (mass 274.6 mg, diameter 9.0 mm). The obtained core tablets (15 g) were coated with a water dispersion containing a mixture (trade name: OPADRY, manufactured by Japan Colorcon) (0.6 g) of hypromellose, titanium oxide, lactose hydrate, macrogol 4000, and triacetin in a coating machine (trade name: HCT-LABO, manufactured by Freund) to give the tablet containing 100 mg of the compound of the formula [I] per tablet.

### Example 6

### Preparation of solid dispersion tablet using HPMCAS and methylcellulose as carrier (preparation by hot-melt extrusion method (small-scale))

A monohydrate (25.9 g) of the compound of the formula [I], HPMCAS (trade name: AQOAT AS-LF, manufactured by Shin-Etsu Chemical Co., Ltd.) (12.5 g) and methylcellulose (trade name: METOLOSE SM-4, manufactured by Shin-Etsu Chemical Co., Ltd.) (2.5 g) were mixed in a bag, and sieved through a sieve with an opening of 710 µm. This mixed powder (5 g) was processed for 5 min with a twin screw extruder (trade name: HAKKE MiniCTW, manufactured by Thermo Fisher Scientific) at a kneading unit barrel temperature 150°C and screw speed 100 rpm to give a cylinder-shaped moldings. This operation was performed twice, and the obtained moldings were milled by a powermill (trade name: new PowerMill, manufactured by OSAKA CHEMICAL Co., Ltd.) at rotating speed 22,000 rpm. The obtained milled granules (3.8 g) were mixed with low-substituted hydroxypropyl cellulose (trade name: L-HPC LH-B1, manufactured by Shin-Etsu Chemical Co., Ltd.) (1.4 g) and magnesium stearate (trade name: Parteck LUB MST, manufactured by Merck) (0.02 g), and then compressed to give a core tablet (mass 224.6 mg, diameter 8.0 mm). The obtained core tablets (2.2 g) were coated with a water dispersion containing a mixture (trade name: OPADRY, manufactured by Japan Colorcon) (12 g) of hypromellose, titanium oxide, lactose hydrate, macrogol 4000, and triacetin together with dummy tablets (300 g) in a coating machine (trade name: HCT-LABO, manufactured by Freund) to give the tablet containing 100 mg of the compound of the formula [I] per tablet.

### Example 7

### Preparation of solid dispersion tablet using HPMCAS and methylcellulose as carrier (preparation by hot-melt extrusion method (large-scale))

A monohydrate (396 g) of the compound of the formula [I], HPMCAS (trade name: AQOAT AS-LF, manufactured by Shin-Etsu Chemical Co., Ltd.) (191 g), methylcellulose (trade name: METOLOSE SM-4, manufactured by Shin-Etsu Chemical Co., Ltd.) (38 g) and magnesium stearate (trade name: Parteck LUB MST, manufactured by Merck) (2 g) were mixed by a V-type diffusion mixer (trade name: V-20, manufactured by TOKUJU CORPORATION). This mixed powder was processed with a dry granulator (trade name: TF-MINI, manufactured by Freund) and a screen mill (trade name: QC-U5, manufactured by Quadro Engineering) to give granules with a specific volume 2.2 mL/g. The obtained granules (579 g) were processed with a twin screw extruder (trade name: Nano-16, manufactured by Leistriz) at a maximum temperature 155°C and screw speed 200 rpm to give a cylinder-shaped moldings. The obtained moldings were milled by an impact mill (trade name: SAMF, manufactured by NARA MACHINERY CO., LTD.) at rotating speed 14000 rpm. The obtained milled granules (197 g) were blended with low-substituted hydroxypropyl cellulose (trade name: L-HPC LH-B1, manufactured by Shin-Etsu Chemical Co., Ltd.) (72 g) and magnesium stearate (trade name: Parteck LUB MST, manufactured by Merck) (1 g), and then compressed to give a core tablet (mass 225.0 mg, diameter 8.0 mm). The obtained core tablets (225 g) were coated with a water dispersion containing a mixture (trade name: OPADRY, manufactured by Japan Colorcon) (7 g) of hypromellose, titanium oxide, lactose hydrate, macrogol 4000, and triacetin in a coating machine (trade name: HCT-LABO, manufactured by Freund) to give the tablet containing 100 mg of the compound of the formula [I] per tablet.

### Comparative Example 1

### Preparation of conventional tablet (small-scale)

A monohydrate (particle size D90 13 µm) (518 g) of the compound of the formula [I] micronized by an impact mill (trade name: SAMF, manufactured by NARA MACHINERY CO., LTD.), lactose hydrate (trade name: Pharmatose 200M, manufactured by DFE Pharma) (103 g) and carmellose calcium (trade name: ECG-505, manufactured by GOTOKU CHEMICAL CO., LTD.) (75 g) were mixed, a solution of hydroxypropyl cellulose (trade name: HPC-L, manufactured by Nippon Soda Co., Ltd.) (25 g) and purified water (425 g) was added thereto, and the mixture was granulated by a high-shear granulator (trade name: FM-VG-10, manufactured by POWREX). The obtained granules were dried to loss on drying 3.5% in a fluid bed granulator (trade name: FD-MP-01, manufactured by POWREX) set to charge air temperature 65°C, and milled by a screen mill with an opening of 610 µm (trade name: QC-U10, manufactured by Quadro Engineering) to give granules. This operation was repeated twice, and the obtained granules (1347 g) and magnesium stearate (trade name: Parteck LUB MST, manufactured by Merck) (8 g) were blended, and then compressed to give a core tablet (mass 145.0 mg, hardness about 70N, diameter 7.5 mm). The obtained core tablets (1088 g) were coated with a water dispersion containing a mixture (trade name: OPADRY, manufactured by Japan Colorcon) (32 g) of hypromellose, titanium oxide, lactose hydrate, macrogol 4000, and triacetin in a coating machine (trade name: HCT-LABO, manufactured by Freund) to give the tablet containing 100 mg of the compound of the formula [I] per tablet.

### Comparative Example 2

### Preparation of conventional tablet (large-scale)

(1) A monohydrate (particle size D90 12 µm) (518 g) of the compound of the formula [I] micronized by an impact mill (trade name: SAMF, manufactured by NARA MACHINERY CO., LTD.), lactose hydrate (trade name: Pharmatose 200M, manufactured by DFE Pharma) (103 g) and carmellose calcium (trade name: ECG-505, manufactured by GOTOKU CHEMICAL CO., LTD.) (75 g) were mixed, a solution of hydroxypropyl cellulose (trade name: HPC-L, manufactured by Nippon Soda Co., Ltd.) (25 g) and purified water (425 g) was added thereto, and the mixture was granulated by a high-shear granulator (trade name: FM-VG-10, manufactured by POWREX). The obtained granules were dried to loss on drying 3.5% in a fluid bed dryer granulator (trade name: FD-MP-01, manufactured by POWREX) set to charge air temperature 65°C, and milled by a screen mill with an opening of 610 µm (trade name: QC-U10, manufactured by Quadro Engineering) to give granules. This operation was repeated 5 times, and the obtained granules (2884 g) were blended with magnesium stearate (trade name: Parteck LUB MST, manufactured by Merck) (16 g) and then compressed to give a core tablet (mass 145.0 mg, diameter 7.5 mm). The obtained core tablets (1088 g) were coated with a water dispersion containing a mixture (trade name: OPADRY, manufactured by Japan Colorcon) (32 g) of hypromellose, titanium oxide, lactose hydrate, macrogol 4000, and triacetin in a coating machine (trade name: HCT-LABO, manufactured by Freund) to give the tablet containing 100 mg of the compound of the formula [I] per tablet.
(2) A monohydrate (particle size D90 12 µm) (518 g) of the compound of the formula [I] micronized by an impact mill (trade name: SAMF, manufactured by NARA MACHINERY CO., LTD.), lactose hydrate (trade name: Pharmatose 200M, manufactured by DFE Pharma) (103 g) and carmellose calcium (trade name: ECG-505, manufactured by GOTOKU CHEMICAL CO., LTD.) (75 g) were mixed, a solution of hydroxypropyl cellulose (trade name: HPC-L, manufactured by Nippon Soda Co., Ltd.) (25 g) and purified water (425 g) was added thereto, and the mixture was granulated by a high-shear granulator (trade name: FM-VG-10, manufactured by POWREX). The obtained granules were dried to loss on drying 3.3% in a fluid bed dryer granulator (trade name: FD-MP-01, manufactured by POWREX) set to charge air temperature 65°C, and sieved by a sieve with an opening of 250 µm to give granules. The obtained granules (433 g), lactose hydrate (trade name: Dilactose S, manufactured by Freund Corporation) (398 g), crystalline cellulose (trade name: CEOLUS UF-711, manufactured by Asahi Kasei Corporation) (720 g), carmellose calcium (trade name: ECG-505, manufactured by GOTOKU CHEMICAL CO., LTD.) (135 g), hydroxypropyl cellulose (trade name: HPC-L, manufactured by Nippon Soda Co., Ltd.) (45 g) and magnesium stearate (trade name: Parteck LUB MST, manufactured by Merck) (10 g) were blended and then compressed to give a core tablet (mass 145.0 mg, diameter 7.5 mm). The obtained core tablets (1088 g) were coated with a water dispersion containing a mixture (trade name: OPADRY, manufactured by Japan Colorcon) (32 g) of hypromellose, titanium oxide, lactose hydrate, macrogol 4000, and triacetin in a coating machine (trade name: HCT-LABO, manufactured by Freund) to give the title tablet containing 25 mg of the compound of the formula [I] per tablet.

### Experimental Example 1

### Dissolution test in neutral media (pH 6.8)

The dissolution profiles of the solid dispersion granules obtained in Example 1 in a pH 6.8 test media (trade name: diluted McIlvaine buffer, manufactured by KANTO CHEMICAL CO., INC.) was evaluated.

The dissolution test was performed using a dissolution tester (trade name: NTR-VS6P, manufactured by Toyama Sangyo Co., Ltd.) that conforms to the Japanese Pharmacopoeia Dissolution Test Method 2 (Paddle Method), at paddle rotating speed 75 rpm, test media amount 450 mL. An amount equivalent to 100 mg of the compound of the formula [I] was added to the test media, and the dissolved amount of the compound of the formula [I] was measured at each sampling point with a spectrophotometer (UV-1600, manufactured by Shimadzu Corporation).

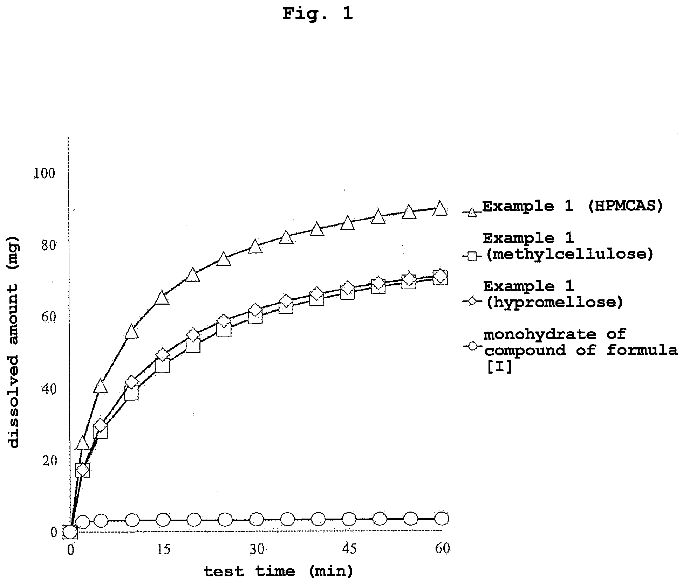

As a comparison control, the dissolution profiles of a monohydrate (particle size D90: 13 µm) crystal of the compound of the formula [I] micronized by an impact mill (trade name: SAMF, manufactured by NARA MACHINERY CO., LTD.) was also evaluated by the same method. The results are shown in Fig. 1.

As shown in Fig. 1, all the solid dispersions of Example 1 showed better solubility than the monohydrate crystals of the compound of the formula [I].

### Experimental Example 2

### Dissolution test in neutral media (pH 6.8)

The dissolution profiles of the solid dispersion granules obtained in Example 2 in a pH 6.8 test media (trade name: diluted McIlvaine buffer, manufactured by KANTO CHEMICAL CO., INC.) was evaluated by a method similar to that in Experimental Example 1.

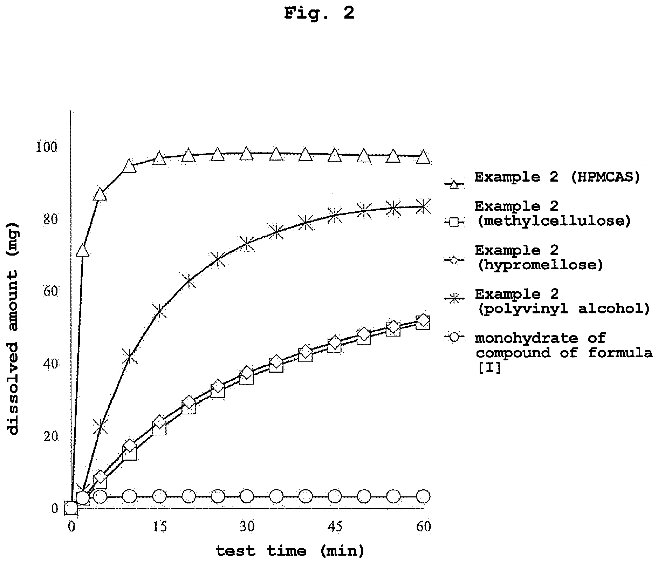

As a comparison control, the dissolution profiles of a monohydrate (particle size D90: 13 µm) crystal of the compound of the formula [I] micronized by an impact mill (trade name: SAMF, manufactured by NARA MACHINERY CO., LTD.) was also evaluated by the same method. The results are shown in Fig. 2.

As shown in Fig. 2, all the solid dispersions of Example 2 showed better solubility than the monohydrate crystal of the compound of the formula [I].

### Experimental Example 3

### Precipitation test in acidic media (pH 1.2)

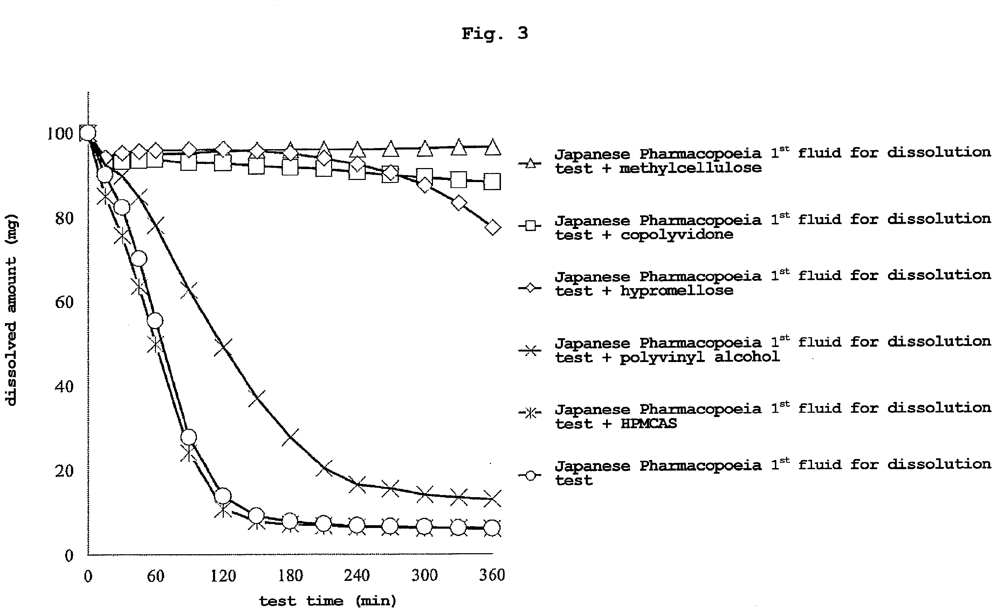

The precipitation behavior of the compound of the formula [I], dissolved in ethanol (concentration: 100 mg/mL), in the following 6 types of test medium was evaluated by the same test method as in Experimental Example 1. The results are shown in Fig. 3.
1) the Japanese Pharmacopoeia 1^{st} fluid for dissolution test (pH 1.2, 450 mL) added with copolyvidone (trade name: Kollidon VA 64, manufactured by BASF) (0.01 g)
2) the Japanese Pharmacopoeia 1^{st} fluid for dissolution test (pH 1.2, 450 mL) added with methylcellulose (trade name: METOLOSE SM-4, manufactured by Shin-Etsu Chemical Co., Ltd.) (0.01 g)
3) the Japanese Pharmacopoeia 1^{st} fluid for dissolution test (pH 1.2, 450 mL) added with hypromellose (trade name: TC-5E, manufactured by Shin-Etsu Chemical Co., Ltd.) (0.01 g)
4) the Japanese Pharmacopoeia 1^{st} fluid for dissolution test (pH 1.2, 450 mL) added with HPMCAS (trade name: AQOAT AS-MF, manufactured by Shin-Etsu Chemical Co., Ltd.) (0.01 g)
5) the Japanese Pharmacopoeia 1^{st} fluid for dissolution test (pH 1.2, 450 mL) added with polyvinyl alcohol (trade name: JL-05E, manufactured by JAPAN VAM & POVAL CO., LTD.) (0.01 g)
6) test media: the Japanese Pharmacopoeia 1^{st} fluid for dissolution test (pH 1.2, 450 mL)

As shown in Fig. 3, the addition of copolyvidone, methylcellulose, or hypromellose significantly suppressed the precipitation of the compound of the formula [I], as compared with the addition of HPMCAS, the addition of polyvinyl alcohol, or without addition of a pharmaceutically acceptable polymer.

### Experimental Example 4

### Dissolution test in neutral media and acidic media

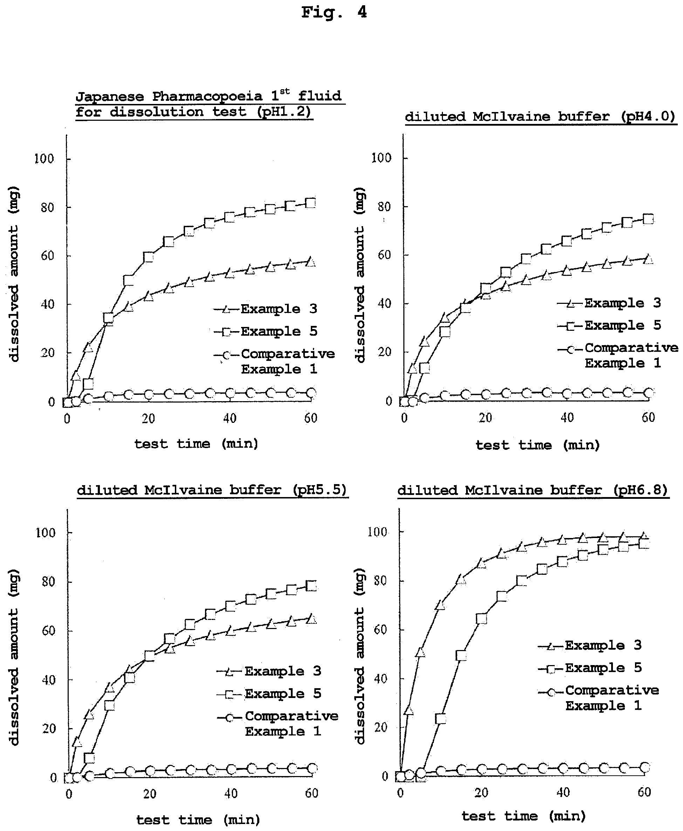

The dissolution profiles of the tablets obtained in Example 3, Example 5 or Comparative Example 1 in the following 4 test medium was evaluated by a method similar to that in Experimental Example 1. The results are shown in Fig. 4.
1) pH 1.2 test media (the Japanese Pharmacopoeia 1^{st} fluid for dissolution test, manufactured by KANTO CHEMICAL CO., INC.) 450 mL
2) pH 4.0 test media (trade name: diluted McIlvaine buffer, manufactured by KANTO CHEMICAL CO., INC.) 450 mL
3) pH 5.5 test media (trade name: diluted McIlvaine buffer, manufactured by KANTO CHEMICAL CO., INC.) 450 mL
4) pH 6.8 test media (trade name: diluted McIlvaine buffer, manufactured by KANTO CHEMICAL CO., INC.) 450 mL

As shown in Fig. 4, the solid dispersion tablets of Example 3 and Example 5 showed better solubility in any of the test medium than the conventional tablet of Comparative Example 1.

### Experimental Example 5

### Stability test of solid dispersion tablet

### Experimental Example 5-1: stability test using solid dispersion tablet of Example 3

The tablets obtained in Example 3 were placed in a glass bottle together with a desiccant under the conditions of 5°C, 25°C/60% RH and 40°C 75% RH, and the bottle was closed and stored for 6 months. Then, the crystalline state was evaluated by Powder X-ray diffractometer (trade name: X'Pert PRO, manufactured by Malvern Panalytical) and differential scanning calorimetry (trade name: DSC Q2000, manufactured by TA instruments). As a controlled comparison, a monohydrate crystal of the compound of the formula [I] was similarly evaluated.

### (Evaluation by powder X-ray diffraction)

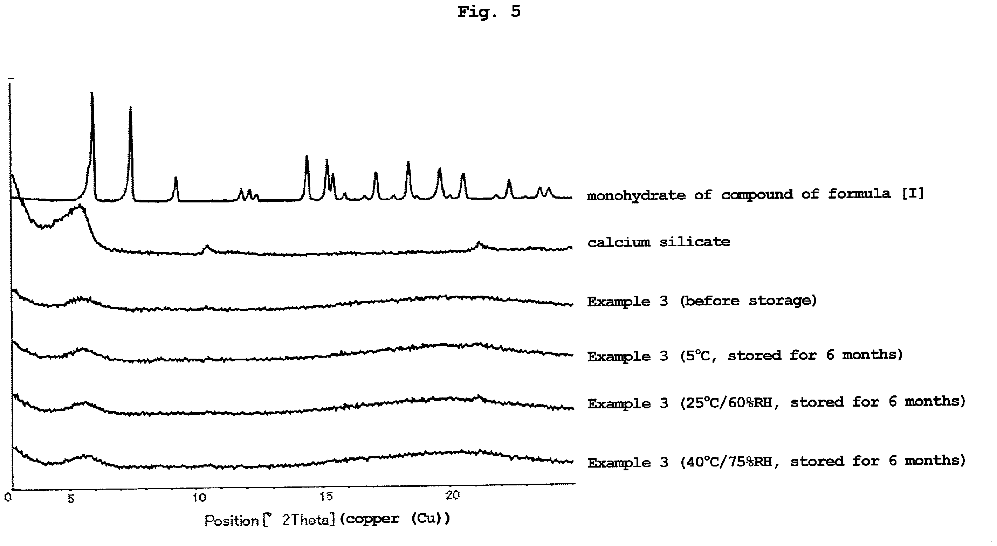

The results of evaluation under the following conditions are shown in Fig. 5.
Synchrotron Radiation: Cu-Kα1/45 kV/40 mA
Counter monochromator: graphite
Scan range: 3 to 25°
Temperature and humidity: 25°C/60%RH

As shown in Fig. 5, since no peak other than the peak derived from calcium silicate, which is an additive component, was observed in the solid dispersion tablet of Example 3, it was confirmed that the compound of the formula [I] was in an amorphous state. In addition, in the absence of changes from before storage under any storage conditions, it was found that the amorphous state was maintained even after storage.

### (Evaluation by differential scanning calorimetry (DSC))

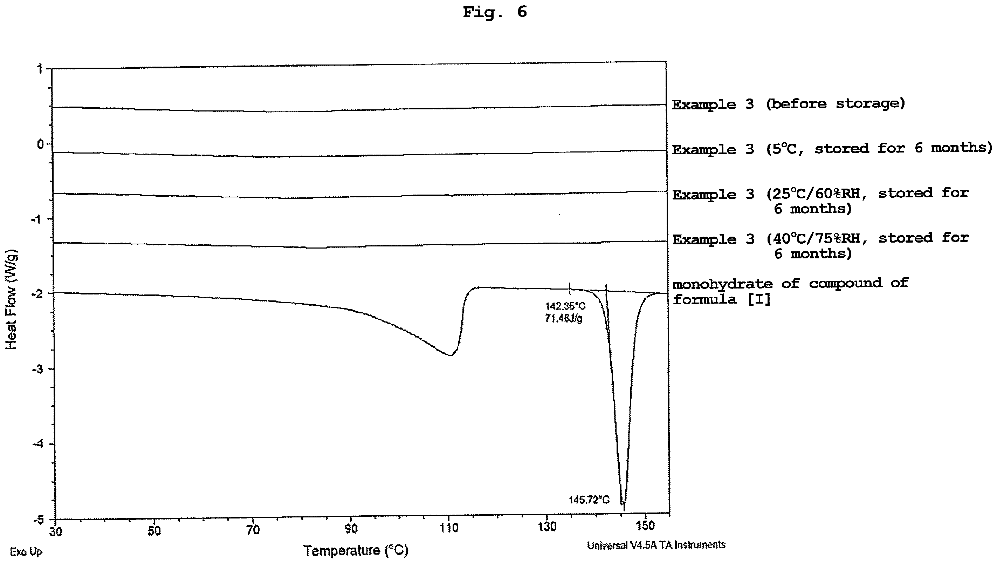

The results of evaluation under the following conditions are shown in Fig. 6.
Sample amount: 5 mg
Container: open aluminum pan (trade name: T zero, manufactured by TA instruments)
Rate of temperature increase: 5°C/min
Temperature range: 25 to 180°C
Atmospheric gas: nitrogen, 50 mL/min
Reference: empty aluminum pan

As shown in Fig. 6, in the monohydrate of the compound of the formula [I], a peak around 100°C due to desorption of water molecules and a peak around 145°C due to melting are observed, whereas the solid dispersion tablet of Example 3 showed a halo pattern, which confirms that the compound of the formula [I] is in an amorphous state. In addition, in the absence of changes from before storage under any storage conditions, it was found that the amorphous state was maintained even after storage.

### Experimental Example 5-2

### Stability test using solid dispersion tablet of Example 7

The tablets obtained in Example 7 were stored under the conditions of 25°C/60% RH and 40°C 75% RH under open conditions for 6 months. Then, the crystalline state was evaluated by differential scanning calorimetry (trade name: DSC Q2000, manufactured by TA instruments). As a controlled comparison, a monohydrate crystal of the compound of the formula [I] was evaluated in the same manner.

### (Evaluation by differential scanning calorimetry (DSC))

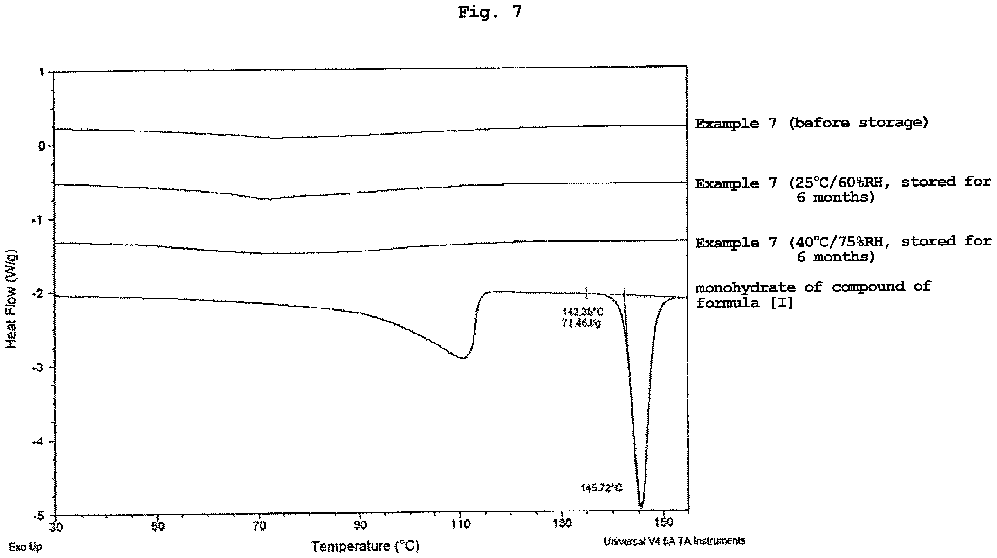

The results of evaluation under the following conditions are shown in Fig. 7.
Sample amount: 5 mg
Container: open aluminum pan (trade name: T zero, manufactured by TA instruments)
Rate of temperature increase: 5°C/min
Temperature range: 25 to 180°C
Atmospheric gas: nitrogen, 50 mL/min
Reference: empty aluminum pan

As shown in Fig. 7, in the monohydrate of the compound of the formula [I], a peak around 100°C due to desorption of water molecules and a peak around 145°C due to melting are observed, whereas the solid dispersion tablet of Example 7 showed a halo pattern, which confirms that the compound of the formula [I] is in an amorphous state. In addition, in the absence of changes from before storage under any storage conditions, it was found that the amorphous state was maintained even after storage.

### Experimental Example 6

### Pharmacokinetics test in dog

### Experimental Example 6-1: comparison with conventional tablet

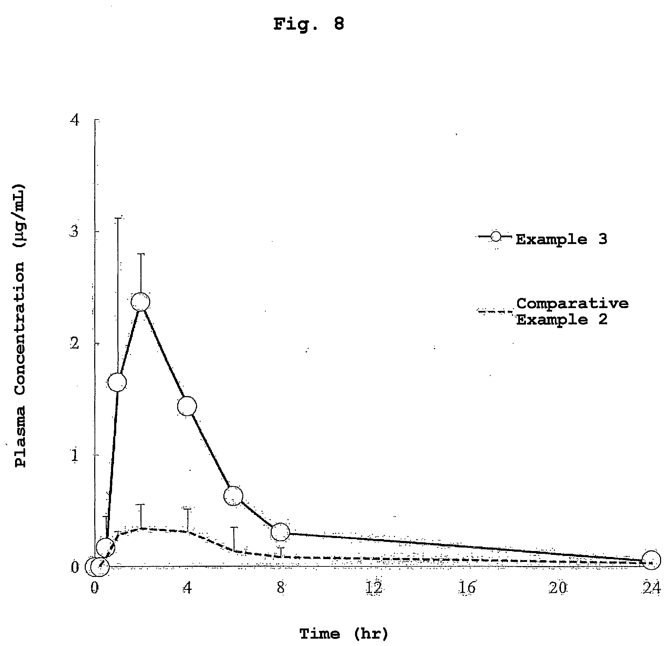

One tablet obtained in Example 3 or Comparative Example 2 (containing an amount equivalent to 100 mg as a compound of the formula [I]) was orally administered to a male beagle dog of the following conditions. The time to maximum plasma concentration (Tₘₐₓ), maximum plasma concentration (Cₘₐₓ), and the area under the plasma concentration curve (AUC₀₋₂₄ₕᵣ) up to 24 hr after administration were calculated from the obtained concentration profile of the obtained plasma. Fig. 8 shows the plasma concentration profile, and Table 1 shows pharmacokinetics parameters.

Dog age: 79 to 81 months
Dog weight: 11.4 to 16 kg
Dietary conditions: fasting from about 17 hr before administration to after blood collection of 4 hr after administration
Gastric pH adjustment: none
Administration method: one tablet is administered by pushing the tablet into the pharynx, followed by addition of about 30 mL of tap water into the oral cavity
Blood collection method: about 1 mL of blood is collected over time from the cephalic vein by using a heparin-treated syringe, and then centrifuged
Plasma concentration measurement time: 0, 0.25, 0.5, 1, 2, 4, 6, 8 and 24 hr after administration
Method for measuring compound concentration in plasma: after deproteinization with acetonitrile, measured using high performance liquid chromatography

As shown in Fig. 8 and Table 1, when the solid dispersion tablet of Example 3 was administered, the Cₘₐₓ value was 6.7 times and the AUC₀₋₂₄ₕᵣ value was 4.3 times that by the administration of the conventional tablet of Comparative Example 2. On the other hand, the Tₘₐₓ value was not significantly different between the solid dispersion tablet of Example 3 and the conventional tablet of Comparative Example 2.

**[Table 1]**

| | Tₘₐₓ (hr) | Cₘₐₓ | | AUC₀₋₂₄ₕᵣ | |
|---|---|---|---|---|---|
| | | (ng/mL) | ratio of Example 3 to Comparative Example 2 | (ng hr/mL) | ratio of Example 3 to Comparative Example 2 |
| Comparative Example 2 | 1.3±0.6 | 0.37±0.21 | - | 2.84±1.16 | - |
| Example 3 | 1.7±0. 6 | 2.47±0.62 | 6.7 times | 12.18±1.67 | 4.3 times |

### Experimental Example 6-2: Comparison between solvent method and hot-melt extrusion method

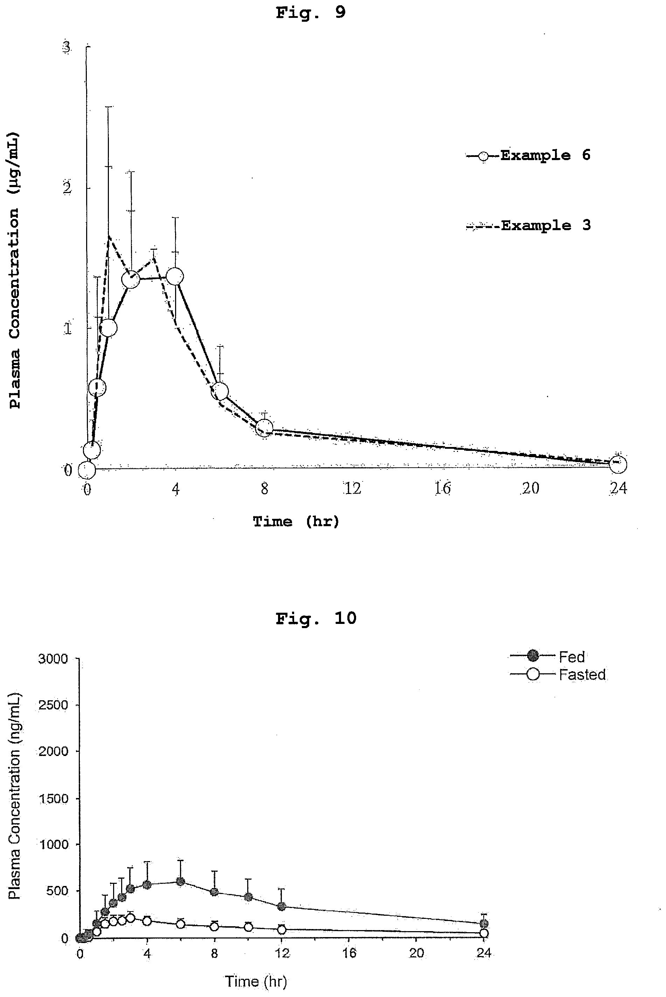

One tablet obtained in Example 3 or Example 6 was orally administered to a male beagle dog under the following conditions. The time to maximum plasma concentration (Tₘₐₓ), maximum plasma concentration (Cₘₐₓ), and the area under the plasma concentration curve (AUC₀₋₂₄ₕᵣ) up to 24 hr after administration were calculated from the obtained concentration profile of the obtained plasma. Fig. 9 shows the plasma concentration profile, and Table 2 shows pharmacokinetics parameters.

Dog age: 90 to 92 months
Dog weight: 11.9 to 16.8 kg
Dietary conditions: fasting from about 17 hr before administration to after blood collection 4 hr after administration
Gastric pH adjustment: implemented (Intravenous administration of pentagastrin 30 min before and 15 min after administration) Administration method: one tablet is administered by pushing the tablet into the pharynx, followed by addition of about 30 mL of tap water into the oral cavity
Blood collection method: about 1 mL of blood is collected over time from the cephalic vein by using a heparin-treated syringe, and then centrifuged
Plasma concentration measurement time: 0, 0.25, 0.5, 1, 2, 3, 4, 6, 8 and 24 hr after administration (measured 3 hr later only when the tablet of Example 3 was administered)
Method for measuring compound concentration in plasma: after deproteinization with acetonitrile, measured using high performance liquid chromatography

As shown in Fig. 9 and Table 2, when the tablet of Example 6 was administered, the Cₘₐₓ value was 0.9 times and the AUC₀₋₂₄ₕᵣ value was 1.0 time that by the administration of the tablet of Example 3, and equivalent oral absorbability was shown. The Tₘₐₓ value was not significantly different between Example 6 and Example 3.

**[Table 2]**

| | Tₘₐₓ (hr) | Cₘₐₓ | | AUC₀₋₂₄ₕᵣ | |
|---|---|---|---|---|---|
| | | (ng/mL) | ratio of Example 6 to Example 3 | (ng hr/mL) | ratio of Example 6 to Example 3 |
| Example 3 | 2.3±1.2 | 1. 95±0. 52 | - | 9.60±0.97 | - |
| Example 6 | 3.0±1.7 | 1.82±0.46 | 0.9 times | 9.68±1.15 | 1.0 time |

### Experimental Example 7

### Pharmacokinetics of the compound of the formula [I] in human clinical test

### Experimental Example 7-1: Clinical test using conventional tablet

Using the tablets obtained in Comparative Example 2, randomized, open-label, and crossover Phase I clinical test was performed to evaluate the influence of diet on the pharmacokinetics of the compound of the formula [I].

On the first day of the first phase (first administration), 14 healthy subjects were randomly assigned at a ratio of 1:1 to receive administration under any of the following conditions, and received a single oral administration of 250 mg of the compound of the formula [I] under the assigned conditions. 250 mg of the compound of the formula [I] was administered using two tablets each containing 100 mg of the compound of the formula [I] and two tablets each containing 25 mg thereof.
(1) Administration under fasting conditions (Fasted): after fasting overnight (at least 10 hr), the compound of the formula [I] is administered without breakfast
(2) Administration under fed conditions (Fed): after fasting overnight (at least 10 hr), the compound of the formula [I] is administered 30 min after the start of ingestion of a high-fat breakfast.

The second phase (second administration) test was performed 7 days after the first administration. The second phase was performed by exchanging the administration conditions of each test subject. That is, the test subjects administered under fasting conditions in the first phase received a single oral administration of 250 mg of the compound of the formula [I] 30 min after the start of ingestion of the high-fat breakfast after fasting overnight (at least 10 hr). The test subjects who received administration under fed conditions in Phase 1 received a single oral administration of 250 mg of the compound of the formula [I] without breakfast after fasting overnight (at least 10 hr).

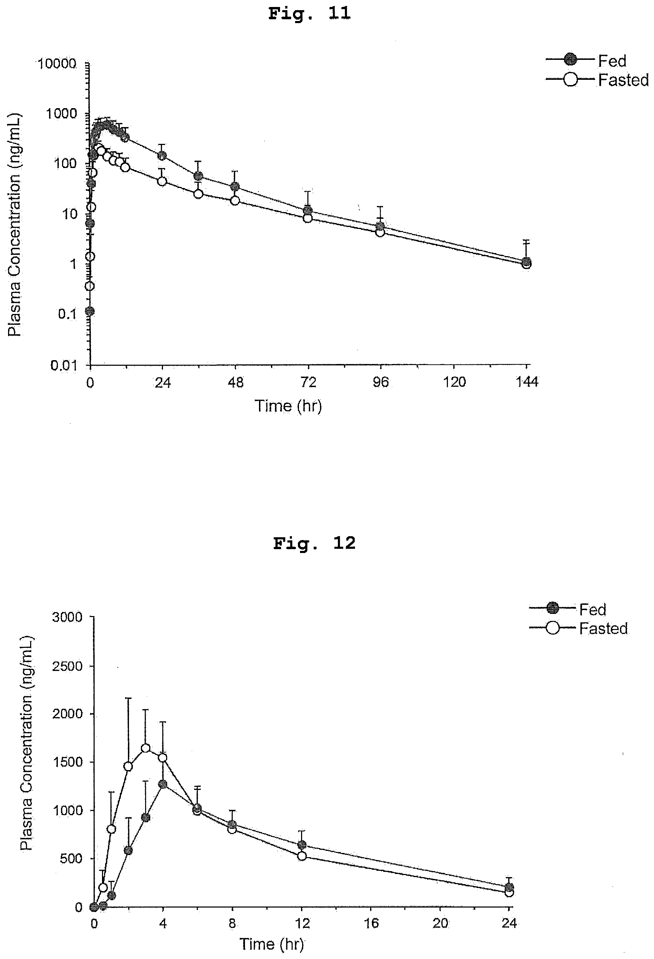

Blood samples were collected from all test subjects 0.5 hr before administration and 0.25, 0.5, 1, 1.5, 2, 2.5, 3, 4, 6, 8, 10, 12, 24, 36, 48, 72, 96 and 144 hr after administration. The plasma concentration of the compound of the formula [I] in the blood samples was measured. From the obtained plasma concentration profile, the maximum plasma concentration arrival time (Tₘₐₓ), maximum plasma concentration (Cₘₐₓ) and plasma concentration area under the curve (AUC_{inf}) were calculated. The plasma concentration profile is shown in Fig. 10 and Fig. 11, and pharmacokinetics parameters are shown in Table 3.

**[Table 3]**

| | Tₘₐₓ (hr) | Cₘₐₓ | | AUC_{inf} | |
|---|---|---|---|---|---|
| | | (ng/mL) (Mean±SD) | ratio of Fed to Fasted | (ng hr/mL) (Mean±SD) | ratio of Fed to Fasted |
| Fasted | 3.00 | 232±67.0 | - | 3540±1710 | - |
| Fed | 5.00 | 708±202 | 3.05 times | 10400±5300 | 2.94 times |

As shown in Fig. 10, Fig. 11 and Table 3, the oral absorbability of the compound of the formula [I] decreased when administered under the fasting conditions as compared with the fed conditions in the clinical test using the conventional tablet of the compound of the formula [I].

### Experimental Example 7-2: Clinical test using solid dispersion tablet

Using the tablets obtained in Example 4, randomized, open-label, and crossover Phase I clinical test was performed to evaluate the influence of diet on the pharmacokinetics of the compound of the formula [I].

On the first day of the first phase (first administration), 14 healthy subjects were randomly assigned at a ratio of 1:1 to receive administration under any of the following conditions, and received a single oral administration of 300 mg of the compound of the formula [I] under the assigned conditions. 300 mg of the compound of the formula [I] was administered using three tablets each containing 100 mg of the compound of the formula [I].
(1) Administration under fasting conditions (Fasted): after fasting overnight (at least 10 hr), the compound of the formula [I] is administered without breakfast
(2) Administration under fed conditions (Fed): after fasting overnight (at least 10 hr), the compound of the formula [I] is administered 30 min after the start of ingestion of a high-fat breakfast.

The second phase (second administration) test was performed 7 days after the first administration. The second phase was performed by exchanging the administration conditions of each test subject. That is, the test subjects administered under fasting conditions in the first phase received a single oral administration of 300 mg of the compound of the formula [I] 30 min after the start of ingestion of the high-fat breakfast after fasting overnight (at least 10 hr). The test subjects who received administration under fed conditions in Phase 1 received a single oral administration of 300 mg of the compound of the formula [I] without breakfast after fasting overnight (at least 10 hr).

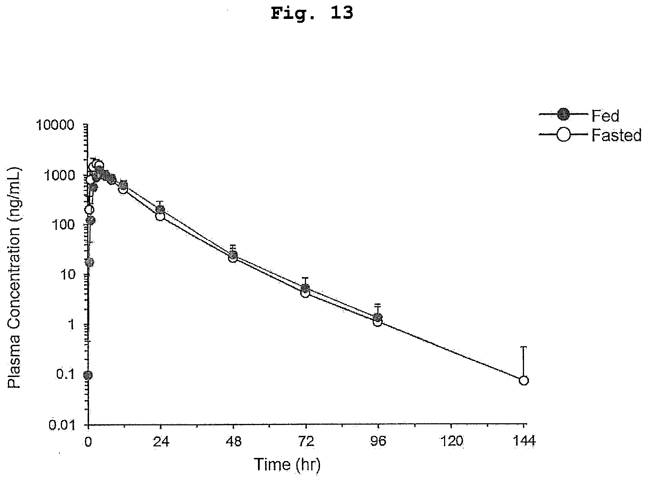

Blood samples were collected from all test subjects 0.5 hr before administration and 0.5, 1, 2, 3, 4, 6, 8, 12, 24, 48, 72, 96 and 144 hr after administration. The plasma concentration of the compound of the formula [I] in the blood samples was measured. From the obtained plasma concentration profile, the maximum plasma concentration arrival time (Tₘₐₓ), maximum plasma concentration (Cₘₐₓ) and plasma concentration area under the curve (AUC_{inf}) were calculated. The plasma concentration profile is shown in Fig. 12 and Fig. 13, and pharmacokinetics parameters are shown in Table 4.

**[Table 4]**

| | Tₘₐₓ (hr) | Cₘₐₓ | | AUC_{inf} | |
|---|---|---|---|---|---|
| | | (ng/mL) (Mean±SD) | ratio of Fed to Fasted | (ng hr/mL) (Mean±SD) | ratio of Fed to Fasted |
| Fasted | 3.00 | 1830±514 | - | 17200±3350 | - |
| Fed | 4.00 | 1320±268 | 0.72 times | 16400±3060 | 0.95 times |

As shown in Fig. 12, Fig. 13 and Table 4, the oral absorbability of the compound of the formula [I] did not show a remarkable difference between administration under fasting and fed conditions in the clinical test using the solid dispersion tablet of the compound of the formula [I].

### [Industrial Applicability]

According to the present invention, a solid dispersion capable of stably maintaining a compound of the formula [I] or a pharmaceutically acceptable salt thereof, or a hydrate thereof in an amorphous state is provided. Consequently, a pharmaceutical preparation containing the compound of the formula [I] with improved pharmacokinetics or a pharmaceutically acceptable salt thereof or a hydrate thereof is provided.

In addition, an amorphous solid dispersion of the compound of the formula [I] according to the present invention is advantageous in that it shows high solubility in some embodiments regardless of the presence or absence of bile acid at the time of administration. Thus, it is less susceptible to the influence of the diet and shows high oral absorbability even when administered under fasting conditions.

This application is based on a patent application No. 2019-038327 filed in Japan (filing date: March 4, 2019).

## Claims

1. An amorphous solid dispersion comprising
(1) a compound represented by the following formula [1]: 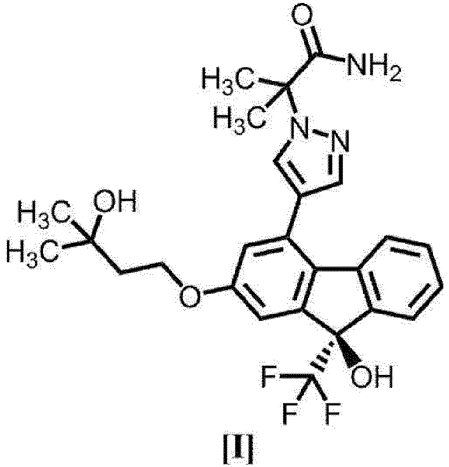 or a pharmaceutically acceptable salt thereof or a hydrate thereof, and
(2) one to four kinds of pharmaceutically acceptable polymers selected from the group consisting of hydroxypropylmethylcellulose acetate succinate, methylcellulose, hypromellose and polyvinyl alcohol.

2. The amorphous solid dispersion according to claim 1, further comprising copolyvidone.

3. The amorphous solid dispersion according to claim 1 or 2, comprising two kinds of pharmaceutically acceptable polymers composed of hydroxypropylmethylcellulose acetate succinate and methylcellulose.

4. The amorphous solid dispersion according to claim 1 or 2, comprising three kinds of pharmaceutically acceptable polymers composed of hydroxypropylmethylcellulose acetate succinate, methylcellulose and polyvinyl alcohol.

5. The amorphous solid dispersion according to claim 3 or 4, wherein a weight ratio of the compound represented by the aforementioned formula [I] or a pharmaceutically acceptable salt thereof or a hydrate thereof, and
hydroxypropylmethylcellulose acetate succinate is within the range of from 1:0.1 to 1:10.

6. The amorphous solid dispersion according to claim 4, wherein a weight ratio of the compound represented by the aforementioned formula [I] or a pharmaceutically acceptable salt thereof or a hydrate thereof, and polyvinyl alcohol is within the range of from 1:0.1 to 1:10.

7. The amorphous solid dispersion according to any one of claims 3 to 6, wherein a weight ratio of the compound represented by the aforementioned formula [I] or a pharmaceutically acceptable salt thereof or a hydrate thereof, and methylcellulose is within the range of from 1:0.05 to 1:1.

8. The amorphous solid dispersion according to any one of claims 1 to 7, wherein the compound represented by the aforementioned formula [I] or a pharmaceutically acceptable salt thereof or a hydrate thereof is a compound represented by the following formula [I-h]:

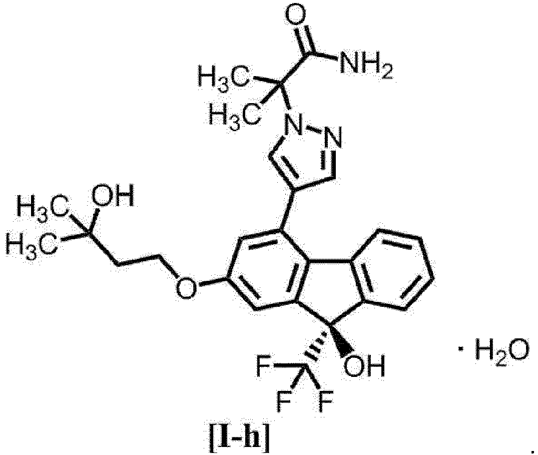

9. The amorphous solid dispersion according to any one of claims 1 to 7, wherein the compound represented by the aforementioned formula [I] or a pharmaceutically acceptable salt thereof or a hydrate thereof is a compound represented by the formula [I].

10. A pharmaceutical composition comprising the amorphous solid dispersion according to any one of claims 1 to 9, and a pharmaceutically acceptable carrier.

11. The pharmaceutical composition according to claim 10, wherein the pharmaceutically acceptable carrier comprises a disintegrant.

12. The pharmaceutical composition according to claim 11, wherein the disintegrant is one to four kinds selected from the group consisting of calcium silicate, croscarmellose sodium, low-substituted hydroxypropyl cellulose and silicified microcrystalline cellulose.

13. The pharmaceutical composition according to claim 11 or 12, further comprising an adsorbent.

14. The pharmaceutical composition according to claim 13, wherein the adsorbent is light anhydrous silicic acid.

15. The pharmaceutical composition according to any one of claims 11 to 14, further comprising a lubricant.

16. The pharmaceutical composition according to claim 15, wherein the lubricant is magnesium stearate.

17. The pharmaceutical composition according to any one of claims 10 to 16, wherein the composition is a film-coated composition.

18. The pharmaceutical composition according to claim 17, wherein the film-coat uses hypromellose.

19. The pharmaceutical composition according to any one of claims 10 to 18, wherein the composition is in the form of a tablet.

20. A method for manufacturing the amorphous solid dispersion according to any one of claims 1 to 9, comprising a step of mixing
(1) a compound represented by the aforementioned formula [I] or a pharmaceutically acceptable salt thereof or a hydrate thereof, and
(2) one to four kinds of pharmaceutically acceptable polymers selected from the group consisting of hydroxypropylmethylcellulose acetate succinate, methylcellulose, hypromellose and polyvinyl alcohol
in a solvent, and dissolving or dispersing them to obtain a mixture,
a step of granulating the aforementioned mixture to obtain a granule, and
a step of drying the aforementioned granule.

21. The manufacturing method according to claim 20, wherein the solvent is acetone.

22. A method for manufacturing the amorphous solid dispersion according to any one of claims 1 to 9, comprising a step of mixing
(1) a compound represented by the aforementioned formula [I] or a pharmaceutically acceptable salt thereof or a hydrate thereof, and
(2) one to four kinds of pharmaceutically acceptable polymers selected from the group consisting of hydroxypropylmethylcellulose acetate succinate, methylcellulose, hypromellose and polyvinyl alcohol
to obtain a mixture, and
a step of hot-melt extrusion processing of the aforementioned mixture.

23. The manufacturing method according to claim 22, wherein the hot-melt extrusion processing step comprises a step of treating in a twin screw extruder.

24. The manufacturing method according to claim 23, wherein the treatment in the twin screw extruder is performed at a temperature of 125°C to 175°C.

25. The manufacturing method according to any one of claims 20 to 24, wherein the pharmaceutically acceptable polymer is composed of hydroxypropylmethylcellulose acetate succinate and methylcellulose.

26. The manufacturing method according to any one of claims 20 to 24, wherein the pharmaceutically acceptable polymer is composed of hydroxypropylmethylcellulose acetate succinate, methylcellulose, and polyvinyl alcohol.

27. The manufacturing method according to any one of claims 20 to 26, wherein the
(1) compound represented by the aforementioned formula [I] or a pharmaceutically acceptable salt thereof or a hydrate thereof, and
(2) hydroxypropylmethylcellulose acetate succinate are mixed at a weight ratio within the range of from 1:0.1 to 1:10.

28. The manufacturing method according to any one of claims 20 to 24, 26 and 27, wherein the
(1) compound represented by the aforementioned formula [I] or a pharmaceutically acceptable salt thereof or a hydrate thereof, and
(2) polyvinyl alcohol
are mixed at a weight ratio within the range of from 1:0.1 to 1:10.

29. The manufacturing method according to any one of claims 20 to 28, wherein the
(1) compound represented by the aforementioned formula [I] or a pharmaceutically acceptable salt thereof or a hydrate thereof, and
(2) methylcellulose
are mixed at a weight ratio within the range of from 1:0.03 to 1:2.

30. The manufacturing method according to any one of claims 20 to 29, wherein the compound represented by the aforementioned formula [I] or a pharmaceutically acceptable salt thereof or a hydrate thereof is a compound represented by the following formula [I-h]:

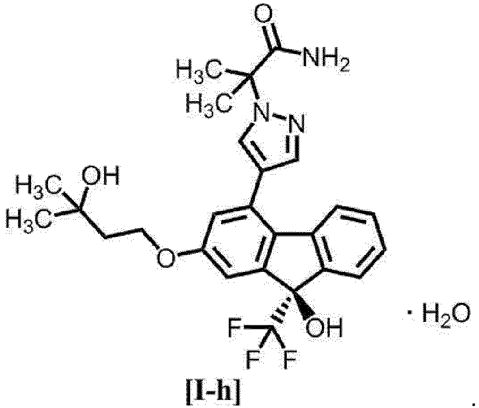

## Patentansprüche

1. Eine amorphe feste Dispersion, umfassend
(1) eine Verbindung, dargestellt durch die folgende Formel [I]: 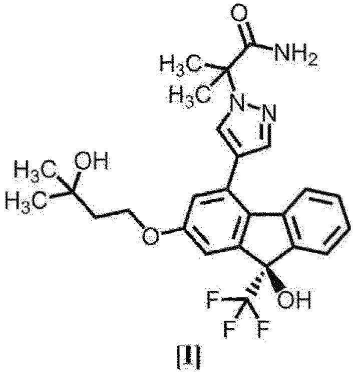 oder ein pharmazeutisch verträgliches Salz davon oder ein Hydrat davon und
(2) eine bis vier Arten pharmazeutisch verträglicher Polymere, ausgewählt aus der Gruppe bestehend aus Hydroxypropylmethylcelluloseacetatsuccinat, Methylcellulose, Hypromellose und Polyvinylalkohol.

2. Die amorphe feste Dispersion nach Anspruch 1, ferner umfassend Copolyvidon.

3. Die amorphe feste Dispersion nach Anspruch 1 oder 2, umfassend zwei Arten pharmazeutisch verträglicher Polymere bestehend aus Hydroxypropylmethylcelluloseacetatsuccinat und Methylcellulose.

4. Die amorphe feste Dispersion nach Anspruch 1 oder 2, umfassend drei Arten pharmazeutisch verträglicher Polymere bestehend aus Hydroxypropylmethylcelluloseacetatsuccinat, Methylcellulose und Polyvinylalkohol.

5. Die amorphe feste Dispersion nach Anspruch 3 oder 4, wobei ein Gewichtsverhältnis der Verbindung, dargestellt durch die vorstehende Formel [I], oder eines pharmazeutisch verträglichen Salzes davon oder eines Hydrats davon und Hydroxypropylmethylcelluloseacetatsuccinat im Bereich von 1:0,1 bis 1:10 liegt.

6. Die amorphe feste Dispersion nach Anspruch 4, wobei ein Gewichtsverhältnis der Verbindung, dargestellt durch die vorstehende Formel [I], oder eines pharmazeutisch verträglichen Salzes davon oder eines Hydrats davon und Polyvinylalkohol im Bereich von 1:0,1 bis 1:10 liegt.

7. Die amorphe feste Dispersion nach einem der Ansprüche 3 bis 6, wobei ein Gewichtsverhältnis der Verbindung, dargestellt durch die vorstehende Formel [I], oder eines pharmazeutisch verträglichen Salzes davon oder eines Hydrats davon und Methylcellulose im Bereich von 1:0,05 bis 1:1 liegt.

8. Die amorphe feste Dispersion nach einem der Ansprüche 1 bis 7, wobei die Verbindung, dargestellt durch die vorstehende Formel [I], oder ein pharmazeutisch verträgliches Salz davon oder ein Hydrat davon eine Verbindung, dargestellt durch die folgende Formel [I-h], ist:

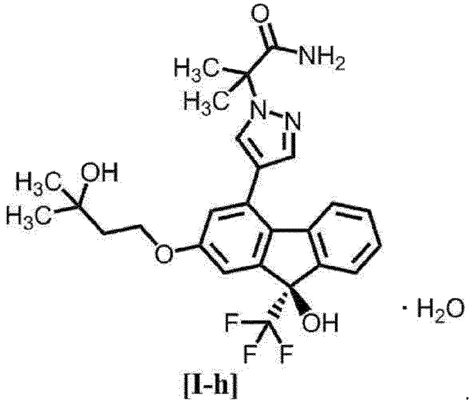

9. Die amorphe feste Dispersion nach einem der Ansprüche 1 bis 7, wobei die Verbindung, dargestellt durch die vorstehende Formel [I], oder ein pharmazeutisch verträgliches Salz davon oder ein Hydrat davon eine Verbindung, dargestellt durch die Formel [I], ist.

10. Ein Arzneimittel, umfassend die amorphe feste Dispersion nach einem der Ansprüche 1 bis 9 und einen pharmazeutisch verträglichen Träger.

11. Das Arzneimittel nach Anspruch 10, wobei der pharmazeutisch verträgliche Träger ein Sprengmittel umfasst.

12. Das Arzneimittel nach Anspruch 11, wobei das Sprengmittel eine bis vier Arten, ausgewählt aus der Gruppe bestehend aus Calciumsilicat, Croscarmellose-Natrium, niedrig substituierter Hydroxypropylcellulose und verkieselter mikrokristalliner Cellulose, ist.

13. Das Arzneimittel nach Anspruch 11 oder 12, ferner umfassend ein Adsorptionsmittel.

14. Das Arzneimittel nach Anspruch 13, wobei das Adsorptionsmittel leichte wasserfreie Kieselsäure ist.

15. Das Arzneimittel nach einem der Ansprüche 11 bis 14, ferner umfassend ein Gleitmittel.

16. Das Arzneimittel nach Anspruch 15, wobei das Gleitmittel Magnesiumstearat ist.

17. Das Arzneimittel nach einem der Ansprüche 10 bis 16, wobei die Zusammensetzung eine filmbeschichtete Zusammensetzung ist.

18. Das Arzneimittel nach Anspruch 17, wobei die Filmbeschichtung Hypromellose verwendet.

19. Das Arzneimittel nach einem der Ansprüche 10 bis 18, wobei die Zusammensetzung in Form einer Tablette vorliegt.

20. Ein Verfahren zur Herstellung der amorphen festen Dispersion nach einem der Ansprüche 1 bis 9, umfassend einen Schritt des Mischens von
(1) einer Verbindung, dargestellt durch die vorstehende Formel [I], oder eines pharmazeutisch verträglichen Salzes davon oder eines Hydrats davon und
(2) einer bis vier Arten pharmazeutisch verträglicher Polymere, ausgewählt aus der Gruppe bestehend aus Hydroxypropylmethylcelluloseacetatsuccinat, Methylcellulose, Hypromellose und Polyvinylalkohol,
in einem Lösungsmittel, und Lösen oder Dispergieren derselben, um ein Gemisch zu erhalten,
einen Schritt des Granulierens des vorstehenden Gemischs, um ein Granulat zu erhalten, und
einen Schritt des Trocknens des vorstehenden Granulats.

21. Das Herstellungsverfahren nach Anspruch 20, wobei das Lösungsmittel Aceton ist.

22. Ein Verfahren zur Herstellung der amorphen festen Dispersion nach einem der Ansprüche 1 bis 9, umfassend einen Schritt des Mischens
(1) einer Verbindung, dargestellt durch die vorstehende Formel [I], oder eines pharmazeutisch verträglichen Salzes davon oder eines Hydrats davon und
(2) einer bis vier Arten pharmazeutisch verträglicher Polymere, ausgewählt aus der Gruppe bestehend aus Hydroxypropylmethylcelluloseacetatsuccinat, Methylcellulose, Hypromellose und Polyvinylalkohol,
um ein Gemisch zu erhalten, und
einen Schritt der Schmelzextrusionsverarbeitung des vorstehenden Gemischs.

23. Das Herstellungsverfahren nach Anspruch 22, wobei der Schmelzextrusionsverarbeitungsschritt einen Schritt der Behandlung in einem Doppelschneckenextruder umfasst.

24. Das Herstellungsverfahren nach Anspruch 23, wobei die Behandlung in dem Doppelschneckenextruder bei einer Temperatur von 125°C bis 175°C durchgeführt wird.

25. Das Herstellungsverfahren nach einem der Ansprüche 20 bis 24, wobei das pharmazeutisch verträgliche Polymer aus Hydroxypropylmethylcelluloseacetatsuccinat und Methylcellulose besteht.

26. Das Herstellungsverfahren nach einem der Ansprüche 20 bis 24, wobei das pharmazeutisch verträgliche Polymer aus Hydroxypropylmethylcelluloseacetatsuccinat, Methylcellulose und Polyvinylalkohol besteht.

27. Das Herstellungsverfahren nach einem der Ansprüche 20 bis 26, wobei
(1) die Verbindung, dargestellt durch die vorstehende Formel [I], oder ein pharmazeutisch verträgliches Salz davon oder ein Hydrat davon und
(2) Hydroxypropylmethylcelluloseacetatsuccinat in einem Gewichtsverhältnis im Bereich von 1:0,1 bis 1:10 gemischt werden.

28. Das Herstellungsverfahren nach einem der Ansprüche 20 bis 24, 26 und 27, wobei
(1) die Verbindung, dargestellt durch die vorstehende Formel [I], oder ein pharmazeutisch verträgliches Salz davon oder ein Hydrat davon und
(2) Polyvinylalkohol
in einem Gewichtsverhältnis im Bereich von 1:0,1 bis 1:10 gemischt werden.

29. Das Herstellungsverfahren nach einem der Ansprüche 20 bis 28, wobei
(1) die Verbindung, dargestellt durch die vorstehende Formel [I], oder ein pharmazeutisch verträgliches Salz davon oder ein Hydrat davon und
(2) Methylcellulose
in einem Gewichtsverhältnis im Bereich von 1:0,03 bis 1:2 gemischt werden.

30. Das Herstellungsverfahren nach einem der Ansprüche 20 bis 29, wobei die Verbindung, dargestellt durch die vorstehende Formel [I], oder ein pharmazeutisch verträgliches Salz davon oder ein Hydrat davon eine Verbindung, dargestellt durch die folgende Formel [I-h], ist:

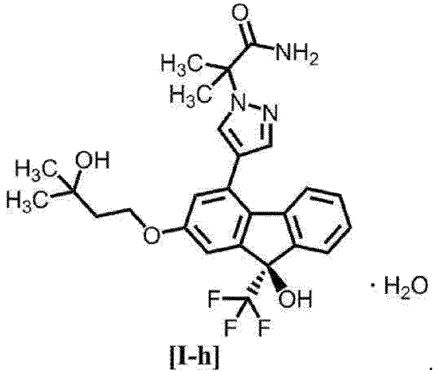

## Revendications

1. Dispersion solide amorphe comprenant
(1) un composé représenté par la formule [I] suivante : 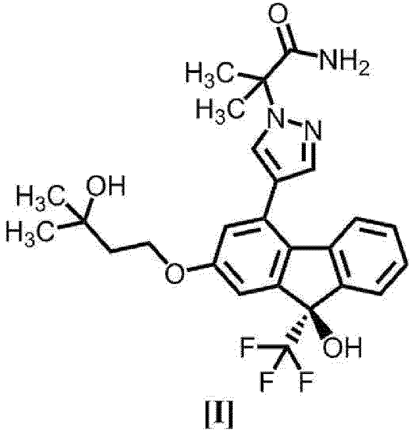 ou un sel pharmaceutiquement acceptable de celui-ci ou un hydrate de celui-ci, et
(2) un à quatre types de polymères pharmaceutiquement acceptables choisis dans le groupe constitué par l'acétosuccinate d'hydroxypropylméthylcellulose, la méthylcellulose, l'hypromellose et l'alcool polyvinylique.

2. Dispersion solide amorphe selon la revendication 1, comprenant en outre de la copolyvidone.

3. Dispersion solide amorphe selon la revendication 1 ou 2, comprenant deux types de polymères pharmaceutiquement acceptables composés d'acétosuccinate d'hydroxypropylméthylcellulose et de méthylcellulose.

4. Dispersion solide amorphe selon la revendication 1 ou 2, comprenant trois types de polymères pharmaceutiquement acceptables composés d'acétosuccinate d'hydroxypropylméthylcellulose, de méthylcellulose et d'alcool polyvinylique.

5. Dispersion solide amorphe selon la revendication 3 ou 4, dans laquelle le rapport en poids du composé représenté par la formule [I] susmentionnée ou d'un sel pharmaceutiquement acceptable de celui ou d'un hydrate de celui-ci, et de l'acétosuccinate d'hydroxypropylméthylcellulose, est situé dans la plage allant de 1/0,1 à 1/10.

6. Dispersion solide amorphe selon la revendication 4, dans laquelle le rapport en poids du composé représenté par la formule [I] susmentionnée ou d'un sel pharmaceutiquement acceptable de celui ou d'un hydrate de celui-ci, et d'alcool polyvinylique, est situé dans la plage allant de 1/0,1 à 1/10.

7. Dispersion solide amorphe selon l'une quelconque des revendications 3 à 6, dans laquelle le rapport en poids du composé représenté par la formule [I] susmentionnée ou d'un sel pharmaceutiquement acceptable de celui ou d'un hydrate de celui-ci, et de la méthylcellulose, est situé dans la plage allant de 1/0,05 à 1/1.

8. Dispersion solide amorphe selon l'une quelconque des revendications 1 à 7, dans laquelle le composé représenté par la formule [I] susmentionnée ou un sel pharmaceutiquement acceptable de celui-ci ou un hydrate de celui-ci est un composé représenté par la formule [I-h] suivante :

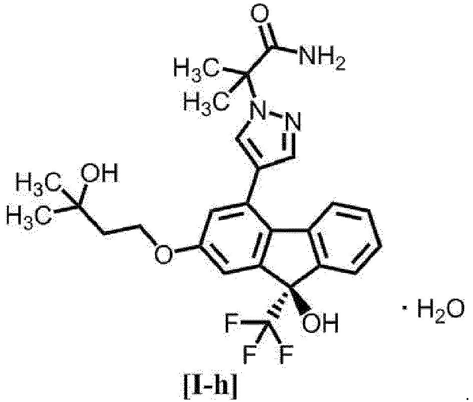

9. Dispersion solide amorphe selon l'une quelconque des revendications 1 à 7, dans laquelle le composé représenté par la formule [I] susmentionnée ou un sel pharmaceutiquement acceptable de celui-ci ou un hydrate de celui-ci est un composé représenté par la formule [I].

10. Composition pharmaceutique comprenant la dispersion solide amorphe selon l'une quelconque des revendications 1 à 9 et un véhicule pharmaceutiquement acceptable.

11. Composition pharmaceutique selon la revendication 10, dans laquelle le véhicule pharmaceutiquement acceptable comprend un agent délitant.

12. Composition pharmaceutique selon la revendication 11, dans laquelle l'agent délitant est un à quatre types choisis dans le groupe constitué par le silicate de calcium, la croscarmellose sodique, l'hydroxypropylcellulose faiblement substituée et la cellulose microcristalline silicifiée.

13. Composition pharmaceutique selon la revendication 11 ou 12, comprenant en outre un agent adsorbant.

14. Composition pharmaceutique selon la revendication 13, dans laquelle l'agent adsorbant est l'acide silicique anhydre léger.

15. Composition pharmaceutique selon l'une quelconque des revendications 11 à 14, comprenant en outre un agent lubrifiant.

16. Composition pharmaceutique selon la revendication 15, dans laquelle l'agent lubrifiant est le stéarate de magnésium.

17. Composition pharmaceutique selon l'une quelconque des revendications 10 à 16, laquelle composition est une composition pelliculée.

18. Composition pharmaceutique selon la revendication 17, dans laquelle le pelliculage utilise de l'hypromellose.

19. Composition pharmaceutique selon l'une quelconque des revendications 10 à 18, laquelle composition est sous la forme d'un comprimé.

20. Méthode pour fabriquer la dispersion solide amorphe de l'une quelconque des revendications 1 à 9, comprenant une étape de mélange
(1) d'un composé représenté par la formule [I] susmentionnée ou d'un sel pharmaceutiquement acceptable de celui-ci ou d'un hydrate de celui-ci, et
(2) d'un à quatre types de polymères pharmaceutiquement acceptables choisis dans le groupe constitué par l'acétosuccinate d'hydroxypropylméthylcellulose, la méthylcellulose, l'hypromellose et l'alcool polyvinylique,
dans un solvant, et de dissolution ou de dispersion de ceux-ci pour obtenir un mélange,
une étape de granulation du mélange susmentionné pour obtenir un granule, et
une étape de séchage du granule susmentionné.

21. Méthode de fabrication selon la revendication 20, dans laquelle le solvant est l'acétone.

22. Méthode pour fabriquer la dispersion solide amorphe de l'une quelconque des revendications 1 à 9, comprenant une étape de mélange
(1) d'un composé représenté par la formule [I] susmentionnée ou d'un sel pharmaceutiquement acceptable de celui-ci ou d'un hydrate de celui-ci, et
(2) d'un à quatre types de polymères pharmaceutiquement acceptables choisis dans le groupe constitué par l'acétosuccinate d'hydroxypropylméthylcellulose, la méthylcellulose, l'hypromellose et l'alcool polyvinylique,
pour obtenir un mélange, et
une étape de traitement d'extrusion à chaud du mélange susmentionné.

23. Méthode de fabrication selon la revendication 22, dans laquelle l'étape de traitement d'extrusion à chaud comprend une étape de traitement dans une extrudeuse double vis.

24. Méthode de fabrication selon la revendication 23, dans laquelle le traitement dans l'extrudeuse double vis est effectué à une température de 125 °C à 175 °C.

25. Méthode de fabrication selon l'une quelconque des revendications 20 à 24, dans laquelle le polymère pharmaceutiquement acceptable est composé d'acétosuccinate d'hydroxypropylméthylcellulose et de méthylcellulose.

26. Méthode de fabrication selon l'une quelconque des revendications 20 à 24, dans laquelle le polymère pharmaceutiquement acceptable est composé d'acétosuccinate d'hydroxypropylméthylcellulose, de méthylcellulose, et d'alcool polyvinylique.

27. Méthode de fabrication selon l'une quelconque des revendications 20 à 26, dans laquelle
(1) le composé représenté par la formule [I] susmentionnée ou un sel pharmaceutiquement acceptable de celui-ci ou un hydrate de celui-ci, et
(2) de l'acétosuccinate d'hydroxypropylméthylcellulose,
sont mélangés en un rapport en poids situé dans la plage allant de 1/0,1 à 1/10.

28. Méthode de fabrication selon l'une quelconque des revendications 20 à 24, 26 et 27, dans laquelle
(1) le composé représenté par la formule [I] susmentionnée ou un sel pharmaceutiquement acceptable de celui-ci ou un hydrate de celui-ci, et
(2) de l'alcool polyvinylique,
sont mélangés en un rapport en poids situé dans la plage allant de 1/0,1 à 1/10.

29. Méthode de fabrication selon l'une quelconque des revendications 20 à 28, dans laquelle
(1) le composé représenté par la formule [I] susmentionnée ou un sel pharmaceutiquement acceptable de celui-ci ou un hydrate de celui-ci, et
(2) de la méthylcellulose,
sont mélangés en un rapport en poids situé dans la plage allant de 1/0,03 à 1/2.

30. Méthode de fabrication selon l'une quelconque des revendications 20 à 29, dans laquelle le composé représenté par la formule [I] susmentionnée ou un sel pharmaceutiquement acceptable de celui-ci ou un hydrate de celui-ci est un composé représenté par la formule [I-h] suivante :
